(19) Europäisches Patentamt
European Patent Office
Office européen des brevets

(11) EP 1 329 506 A1

# (12) EUROPEAN PATENT APPLICATION

(43) Date of publication:
**23.07.2003 Bulletin 2003/30**

(51) Int Cl.[7]: **C12N 15/10**, C12Q 1/68

(21) Application number: **02447009.8**

(22) Date of filing: **18.01.2002**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Cypro S.A.**
**1070 Brussel (BE)**

(72) Inventors:
- **Stordeur, Patrick**
**1190 Bruxelles (BE)**
- **Goldman, Michel**
**1180 Bruxelles (BE)**

(74) Representative: **De Clercq, Ann et al**
**De Clercq, Brants & Partners cv.,**
**Edgard Gevaertdreef 10a**
**9830 Sint-Martens-Latem (BE)**

Remarks:
- The sequence listing, which is published as annex to the application documents, was filed after the date of filing. The applicant has declared that it does not include matter which goes beyond the content of the application as filed.
- Claims 14 to 31 are deemed to be abandoned due to non-payment of the claims fees (Rule 31 (2) EPC).

## (54) Method to quantify in vivo RNA levels

(57) The invention in particular relates to a method for the quantification of *in vivo* RNA from a biological sample comprising the steps of: collecting said biological sample in a tube comprising a compound inhibiting RNA degradation and/or gene induction; forming a precipitate comprising nucleic acids; separating said precipitate from the supernatant; dissolving said precipitate using a buffer, forming a suspension; isolating nucleic acids from said suspension using an automated device; dispersing/distributing a reagent mix for RT-PCR using an automated device; dispersing/distributing the isolated nucleic acids within the dispersed reagent mix using an automated device, and, determining the *in vivo* levels of transcripts using the nucleic acid/RT-PCR reagent mix in an automated setup.The present invention also relates to the quantification of DNA from a biological sample. The present invention further elucidates a kit for isolating quantifiable nucleic acids from a biological sample. Applications of the method according to present invention are also disclosed.



Printed by Jouve, 75001 PARIS (FR)

## Description

### Technical field

The present invention relates to a new nucleic acid analysis method in particular to determine the correct *in vivo* levels of nucleic acid transcripts in biological samples.

### Background art

Deoxyribonucleic acid (DNA), and ribonucleic acid (RNA) are employed in a wide variety of research, medical, diagnostic and industrial processes. The variety of uses for extracted and purified DNA and RNA from disparate sources is rapidly increasing with the advent of biotechnology e.g. for the production of recombinant proteins.
Alternatively, nucleic acid sequences can be employed for diagnostic purposes. For example they can be used to detect the presence of a specific biological agent such as pathogens, viruses or to determine abnormal metabolic changes. With a biological agent is meant all types of agents carrying nucleic acids. Nucleic acid analysis may allow to identify genetic and familial disorders, genetic aberrations and allow to prove identity. Also cellular states (induction of genes, differentiation, etc.) can be identified by visualizing nucleic acid sequences.
In some cases only a qualitative analysis is necessary determining the absence or presence of a specific nucleic acid sequence and/or biological agent. In other cases, real transcript levels need to be determined. Indeed, certain diseases are characterized by the lowered or the increased level of gene expression; certain cell types can only be identified by evaluating the transcript content.
Until now, many tools are available enabling the person, skilled in the art, to perform an isolation of nucleic acids from different biological samples. The collection of a biological sample is the first step in many molecular assays used to study their nucleic acid content.
A major challenge in this type of testing, however, is the instability of RNA *in vitro* especially when the detection of low-level RNA or unstable RNA is aimed at. Even the degradation of only a small fraction of the RNA may change the interpretation of the *in vivo* levels. Some transcripts are known to be present at low copy in a cell; other transcripts have an "AU-rich" sequence in their 3' end promoting their fast degradation by endogenous RNAses. Studies have shown that RNA rapidly degrades significantly within hours after sample collection. Furthermore, certain species of RNA, through the process of gene induction, increase once the sample is collected. Both RNA degradation and *in vitro* gene induction can lead to an under- or over-estimation of the *in vivo* gene transcript number.
Until now, many methods exist to isolate RNA from biological samples. Some allow even the determination of low-level transcripts out of a pool of transcripts. Nevertheless, none of them provide the possibility to determine real *in vivo* levels. With 'real *in vivo* levels' is meant the level(s) of transcript(s) present in the biological agent at the time of the sampling. Storage of biological samples leads to incorrect mRNA levels. Indeed, in practice, the analysis of fresh sample is not feasible as the place of sampling and the place of RNA analysis is located differently.
Recently, PreAnalytiX (a joint venture between Becton Dickinson and Qiagen) has put its first product PAXgene™ Blood RNA System on the market. The PAXgene™ Blood RNA System (also referred to as the Qiagen method) is an integrated and standardized system for the collection and stabilization of whole blood specimens and isolation of cellular RNA. According to PreAnalytiX, in the PAXgene™ Blood RNA System, blood is collected directly into PAXgene™ Blood RNA Tubes and RNA is subsequently isolated using the PAXgene™ Blood RNA Kit. Using this system intact cellular RNA can be retrieved from whole blood.
The PAXgene™ Blood RNA Tube is a plastic, evacuated tube, for the collection of whole blood and stabilization of the cellular RNA profile. The tubes contain an additive (a proprietary blend of reagents) that stabilizes cellular RNA and may eliminate *ex vivo* induction of gene transcription and prevents the drastic changes in the cellular RNA expression profiles that normally take place *in vitro.* RNA is then isolated using silica-gel-membrane technology supplied in the PAXgene™ Blood RNA Kit. According to PreAnalytiX, the resulting RNA accurately represents the expression profile *in vivo* and is suitable for use in a range of downstream applications. According to the supplier, accurate quantification of gene transcripts is possible using this system. A major disadvantage of this PAXgene™ Blood RNA System is that respective PAXgene™ Blood RNA Tube needs to be combined with the PAXgene™ Blood RNA Kit (see instruction manual of the PAXgene™ Blood RNA Tubes). This obliged combination, however, limits further improvement of the system.

### Objects of the invention

Although the PreanalytiX (or PAXgene™ Blood RNA) System points towards the fact that the PAXgene™ Blood RNA Tubes can only be combined with the PAXgene™ Blood RNA Kit, the inventors of the present invention aim to improve the suggested system. In addition, the inventors aim to develop a new method allowing the characterization of real *in vivo* transcript levels. In this way, also correct *in vivo* levels of low-level or unstable transcripts can be determined.

These aims have been met by following embodiments.

The present invention relates to a method for the quantification of *in vivo* RNA from a biological sample comprising the steps of:

(a) collecting said biological sample in a tube comprising a compound inhibiting RNA degradation and/or gene induction,
(b) forming a precipitate comprising nucleic acids,
(c) separating said precipitate of step (b) from the supernatant,
(d) dissolving said precipitate of step (c) using a buffer, forming a suspension,
(e) isolating nucleic acids from said suspension of step (d) using an automated device,
(f) dispersing/distributing a reagent mix for RT-PCR using an automated device,
(g) dispersing/distributing the nucleic acids isolated in step (e) within the dispersed reagent mix of step (f) using an automated device, and,
(h) determining the *in vivo* levels of transcripts using the nucleic acid/RT-PCR reagent mix of step (g) in an automated setup.

Inhibition of RNA degradation and/or gene induction at the moment of the biological sampling is crucial in order to retrieve a pool of RNAs which can be used to determine the *in vivo* transcript levels. It is true that cellular RNA can be purified using the PAXgene™ Blood RNA System; nevertheless, the present inventors proved that real *in vivo* levels can not be measured using this system 'as such' (see example 3).

The present inventors give proof in the present invention that the *in vivo* levels of nucleic acid transcripts can only be measured/determined/quantified when starting from a pool of RNA prepared from a stabilized biological sample, using a compound inhibiting extra- and/or intracellular RNA degradation and/or gene induction; whereby the isolation of the nucleic acids is performed using an automated device, whereby the reagent mix and the isolated nucleic acids, used for the RT-PCR reaction, are dispersed using an automated device, and whereby the determination of the transcript levels is performed in an automated setup. According to the present invention, only this approach allows to quantify *in vivo* RNA in a reproducible manner. The number of steps performed in said method is reduced to a minimum in order to avoid errors. An 'error' may be a pipetting-, a handling-, a procedural- and/or a calculation error or any error which can be made by a person skilled in the art. In this respect, the present inventors suggest in the present invention to perform the RT and the PCR reaction in one step. The method of the present invention will even be more accurate when combining more intermediate steps. For example, in the method of the present invention steps (a) and (b) can be combined.

According to the present invention, the dispersion of the nucleic acids (step (g)) may be performed after, before or simultaneously with the dispersion of the reagent mix needed for RT-PCR (step (f)).

According to the method of present invention, no OD measurements need to be performed, eliminating the errors made in the calculation of the nucleic acid concentration. Contrarily, using the PAXgene™ Blood RNA kit OD measurements need to be made. This illustrates again that the method according to present invention is a more reliable and accurate method compared to the latter system. This better accuracy of the present invention is illustrated by the reproducibility studies presented in table 4.

According to the present invention, when dissolving the formed precipitate in step (d) of the method according to the present invention, the obtained suspension can be used in combination with an RNA extraction method and an analyzing method which are fully automated. It is only this combination which allows to optimize accuracy and reproducibility of the performed method and which allows to determine real *in vivo* RNA levels. As the brochure of the PAXgene™ Blood RNA System describes that the corresponding tubes can not be used in combination with other isolation methods, and no detailed information is available describing the different compositions of the kit, it is not obvious for a person skilled in the art to use parts of this PAXgene™ Blood RNA System and develop a new method therefrom.

There exist only few commercial systems which allow to isolate RNA fully automatically. Examples of such automated nucleic acid extractors are: the MagNA Pure LC Instrument (Roche Diagnostics), The AutoGenprep 960 (Autogen), the ABI PrismTM 6700 Automated Nucleic Acid Workstation (Applied Biosystems), WAVE® Nucleic Acid Analysis System with the optional WAVE® Fragment Collector FCW 200 (Transgenomic) and the BioRobot 8000 (Qiagen).

The present invention points towards the fact that for all these systems it is essential to start with material which is as fresh as possible or which is stabilized in order to allow the determination of real *in vivo* transcript levels. The problem for all these systems is that the biological sample is collected and brought to the laboratory in tubes that contain no or only a conventional additive, so that mRNA can still be rapidly degraded. Consequently, mRNA quantification using these methods will undoubtedly lead to the quantification of the transcripts present in the tube, but this quantification does not represent the transcript levels present in the cells/biological agent at the moment of sampling. Experimental evidence of this is provided in figure 2.2 of example 2 of the present invention.

With the term 'quantification' is meant accurate and reproducible determination of RNA copy numbers; but it is trivial for a person skilled in the art that also qualitative or semiquantitative studies can be performed using RNA isolated via a method as described by the present invention.

The definition 'transcript' is not limited to messenger RNA (mRNA) but also relates to other types of RNA molecules known to exist by a person skilled in the art. According to the method of the present invention mRNA as well as total RNA can be extracted. This allows to get a correct estimation of the *in vivo* nuclear RNA, providing a powerful tool to evaluate gene transcription.

With 'biological sample' is meant a sample containing nucleic acids/biological agents such as clinical (e.g. cell fractions, whole blood, plasma, serum, urine, tissue, cells, etc.), agricultural, environmental (eg. soil, mud, minerals, water, air), food (any food material), forensic or other possible samples. With 'whole blood' is meant blood such as it is collected by venous sampling, i.e. containing white and red cells, platelets, plasma and eventually infectious agents; the infectious agents may be viral, bacterial or parasitical. The clinical samples may be from human or animal origin. The sample analyzed can be both solid or liquid in nature. It is evident when solid materials are used, these are first dissolved in a suitable solution, which could be the RNAlater reagent sold by Qiagen. According to the invention, this solution is not always a real "buffer" with at least two well balanced components. It may be a strong hypotonic solution such as NaCl alone or an extraction solution such as with alcohol.

The term 'nucleic acid' refers to a single stranded or double stranded nucleic acid sequence, said nucleic acid may consist of deoxyribonucleotides (DNA) or ribonucleotides (RNA), RNA/DNA hybrids or may be amplified cDNA or amplified genomic DNA, or a combination thereof. A nucleic acid sequence according to the invention may also comprise any modified nucleotide known in the art.

According to the present invention, the nucleic acid may be present extra- or intracellularly in the biological sample.

The 'separation' of the precipitate from the supernatant in step (c) of present method can be performed via centrifugation, filtration, absorption or other means known by a person skilled in the art. Said precipitate may include cells, cell/debris, nucleic acids or a combination thereof. The basis of the concept is to stop the nucleic-acid-containing-agent (or biological agent) from having contact with external sources/pulses/signals. This can be performed by fixing, lysing and/or disintegrating the nucleic-acid-containing-agent, or by any other means known by a person skilled in the art.

The buffer used in step (d) of the method of present invention may be a buffer to dissolve the precipitate obtained in step (c) of said method. This buffer may have additional effects such as lysis or further lysis of the nucleic-acid-containing-agent.

The 'automated device' used may be an automated pipetting device or another automated device known by a person skilled in the art suitable for carrying out the indicated actions.

With a 'reagent mix for RT-PCR' is meant all reagents needed for a simultaneous RT and PCR reaction (with the exception of the oligonucleotides when explicitly mentioned). According to the present invention, 'oligonucleotides' may comprise short stretches of nucleic acids as found in for example primers or probes. According to the present invention, the *in vivo* levels of the nucleic acids can be determined using real-time PCR or by any method allowing the determination of real *in vivo* RNA levels. According to the present invention, this method can be used in combination with micro-arrays or Rnase protection assays.

As pointed out before, storage of biological samples such as blood leads to incorrect mRNA levels. Indeed, in practice, the analysis of fresh sample is not feasible as the place of sampling and the place of RNA analysis is located differently. The method according to the present invention allows to transport biological samples without any effect on their in *vivo* transcript content. Transport of the biological sample can be performed after step (a) or step (b) in the method of the present invention.

Usually, when using blood samples, red blood cells are preferentially eliminated before the nucleic acids are isolated. Red blood cells are rich in hemoglobin and their presence results in the production of highly viscous lysates. Therefore, removal of these allows to isolate nucleic acids in a more improved fashion. However, in the method of the present invention, this step is eliminated as an insoluble precipitate is immediately formed comprising the nucleic acids, separating these from all other components of the biological sample. This illustrates that, in addition to other advantages, the method of the present invention is a superior method in comparison with most prior art methods.

The present invention suggests to apply the PAXgene™ Blood RNA Tubes in the present method. These contain an additive that stabilizes cellular RNA and may eliminate *ex vivo* induction of the gene transcription. No detailed information is provided describing the content of this additive. The brochure refers to patent US 5,906,744 for this purpose. Nevertheless, the tube described in this patent allows a person skilled in the art to prepare nucleic acids from plasma. According to the present inventors, the content as described in US 5,906,744 does not relate to the real content

of the PAXgene™ Blood RNA Tube.

According to the present inventors the content of these tubes may contain a quaternary amine surfactant. Therefore, according to the present invention, a quaternary amine surfactant may be used in step (a) of the method of the present invention. The use of a quaternary amine surfactant in order to stabilize nucleic acids in a biological sample has been previously described in US5,010,183. This patent provides a method for purifying DNA or RNA from a mixture of biological materials. Said method comprises the step of adding a cationic detergent to a mixture containing the RNA or DNA in an amount sufficient to dissolve cells, solubilize any contaminating proteins and lipids in the mixture, and form insoluble hydrophobic complex between the nucleic acid and the detergent. The complex which comprises the RNA or DNA with the detergent thus becomes separated from the solubilized contaminants. In a more recent patent, the same inventors stated that the use of the surfactant, as described in US 5,010,183, and other commercially available surfactants results in inefficient precipitation of RNA and incomplete lysis of blood cells. As there was a need for improved cationic surfactants for this purpose, the inventors of US 5,010,183 searched for a novel method for isolating RNA from a biological sample, including blood, involving the use of an aqueous, cationic surfactant solution comprising a selected quaternary amine (US 5,985,572). The synthesis of the different possible surfactants, that can be used in any methods of the present invention, can be performed according to the instructions as published in above cited or related patents. One example of a quaternary amine which can be used in the method of the present invention is tetradecyltrimethyl-ammonium oxalate. (US 5,985,572). Alternatively, said cationic detergent may be Catrimox-14™ (US5,010,183) as shown in the example 2 of the present invention.

According to the present invention, said compound of step (a) in any method of the present invention may be a compound inhibiting RNA degradation and/or gene induction as found in a PAXgene™ Blood RNA Tube.

The tube which can be used to collect the biological sample depends on the sample taken. For example, blood can be collected in any tube. Therefore, in step (a) of the method according to the present invention, said tube may be an open or a closed blood collecting tube. Nevertheless, preferably a closed tube is used in order to prevent blood splatter, blood leakage and potential exposure to blood borne pathogens. A Hemogard™ closure may be used for this purpose (Becton Dickinson). Furthermore, blood is drained inside the PAXgene™ Blood RNA Tube by vacuum, so that the taken volume is always the same, allowing a "standardized sample volume".

According to the present invention, said buffer used in step (d) of the method of the present invention may be a guanidine-thiocyanate-containing buffer.

In the examples of the present invention the precipitate formed in the PAXgene™ Blood RNA Tubes is dissolved in the lysis buffer as provided by the MagNA Pure LC mRNA Isolation Kit I (Roche Diagnostics, Molecular Biochemicals). Therefore, it is suggested in the present invention that one of the possible buffers which may be used in the method of the present invention is a guanidine-thiocyanate-containing lysis buffer as provided by MagNA Pure LC mRNA Isolation Kit I (Roche Diagnostics, Molecular Biochemicals).

The MagNA Pure LC mRNA Isolation Kit I (Roche Diagnostics, Molecular Biochemicals) is especially designed for use on the MagNA Pure LC Instrument, to guarantee the isolation of high quality and undegraded RNA from whole blood, white blood cells, and peripheral blood lymphocytes. According to its product description, obtained RNA is suitable for highly sensitive and quantitative LightCycler RT-PCR reactions, as well as for standard block cycler RT-PCR reactions, Northern blotting and other standard RNA applications. Nevertheless, the present inventors proved that the use of this method 'as such' could not result in the determination of correct transcript levels. The present inventors showed that there is a need to stabilize the RNA prior to the RNA isolation (see example 2). The present invention describes the unique combination of the use of RNA stabilizing compounds and an automated isolation/analysis procedure.

According to the present invention, once the precipitate of step (d) is dissolved in the lysis buffer as provided by MagNA Pure LC mRNA Isolation Kit I, the method of the present invention may follow the procedure as described for the MagNA Pure LC mRNA Isolation Kit I. After the samples are lysed through the presence of a chaotropic salt in the lysis buffer, streptavidin-coated magnetic particles are added together with biotin-labeled oligo-dT, and the mRNA binds to the surface of the particles. This is followed by a DNase digestion step. mRNA is then separated from unbound substances using a magnet and several washing steps. Finally, the purified mRNAs are eluted. This isolation kit allows the automated isolation of pure mRNA as a "walk away" system. It allows to isolate mRNA of high quality and integrity suitable for all major downstream applications regarding gene expression analysis. Different protocols are offered depending on the sample material used. The samples may be set directly on the MagNA pure LC Instrument stage. Nevertheless, when using whole blood, cells present in the samples are preferentially lysed manually. mRNA isolation may then be postponed or directly further processed on the instrument.

The present inventors prove in the present examples that the use of the MagNA Pure LC Instrument (Roche Diagnostics, Molecular Biochemicals) as automated device in step (e), step (f) and/or step (g) of the method according to the present invention leads to the production of a pool of RNA which can be used to determine exact/real *in vivo* levels of transcripts. RNA-capturing beads such as magnetic beads, coated with oligo-dT via a streptavidin-biotin system or an equivalent system, may be applied in the method of the present invention in order to separate mRNA from

the cellular debris.

Alternatively, according to the present invention other automated devices may be used such as the ABI PrismTM 6700 Automated Nucleic Acid Workstation (Applied Biosystems) or any other automated device that can be used for this purpose.

In the brochure of the MagNA pure LC mRNA Isolation Kit I (Cat No 3 004 015) no compositions of the buffers used in this kit are mentioned in detail. There is only a reference to the presence of guanidinium thiocyanate for safety purposes (page 7). There is no mentioning of additional compounds in this buffer. Therefore it is not obvious for a person skilled in the art to assume that the buffer as provided by this kit would allow to dissolve the pellet obtained by the method of the PAXgene™ Blood RNA Tubes. In addition, a person skilled in the art would not combine both methods based on the information provided by the PAXgene™ Blood RNA Tubes brochure stating that these tubes can only be combined with the corresponding PAXgene™ Blood RNA Kit (page 3, see limitations of the system; page 6, see ordering information). Furthermore, there is also no urge for further optimization as it is claimed by respective companies that high quality of RNA can be obtained using the MagNA pure LC mRNA Isolation Kit I or the PAXgene™ Blood RNA System.

As pointed out above, when using blood samples, red blood cells are preferentially lysed after step (a) in the method of the present invention. In the design of the MagNA Pure LC mRNA Isolation Kit I (Roche Diagnostics, Molecular Biochemicals) there is a possibility to lyse and eliminate red blood cells, before mRNA isolation from white blood cells. Nevertheless, because of this step, samples can not be treated fast enough to avoid mRNA degradation. The inventors decided to use PAXgene™ Blood RNA Tube in conjunction with the MagNA Pure mRNA Isolation Kit on the MagNA Pure Instrument. Using the PAXgene™ Blood RNA Tubes provides a precipitate of nucleic acids that is not supposed to be soluble in the lysis buffer of the MagNA Pure mRNA Isolation Kit. Despite of this, the inventors found that it is actually possible. Following this observation, the inventors combined the use of the PAXgene™ Blood RNA Tubes with the use of an automated RNA isolation system. The inventors found surprisingly that this combination is possible and that this combination provides a powerful technique for the accurate mRNA quantification from biological samples.

The RNA isolated using the method according to the present invention is ready for use in a wide range of downstream applications, including for instance nucleic acid amplification technologies, such as RT-PCR and NASBA®, Expression-array and expression-chip analysis, Quantitative RT-PCR, including TaqMan® technology, cDNA synthesis, RNase and S1 nuclease protection, Northern, dot, and slot blot analysis and primer extension.

The present inventors showed in the example 2 and example 3 of the present invention that the use of a compound inhibiting RNA degradation and/or gene induction in conjunction with an automated RNA isolation and an automated analysis method such as real time PCR allows the determination of *in vivo* levels of transcripts. Nevertheless, according to present invention analysis methods other than real-time PCR may be applied as long as they are provided in an automated setup.

A main advantage of the method according to the present invention, is the fact that by using this method small sample volumes can be analyzed. This is of prime importance when only small volumes are available, for example when analyzing neonatal blood samples or in cases of high blood loss. According to the present concept RNA quantification may be performed using a biological sample as small as 100 μl. The analysis of RNA from a sample as small as 100 μl is not possible with the Qiagen kit (PAXgen™ Blood RNA System) which requires a larger volume of blood (2.5 ml following the kit handbook).

The present invention also relates to a method for the quantification of *in vivo* RNA from a biological sample comprising the steps of:

(a) collecting a biological sample in the PAXgene™ Blood RNA Tube,
(b) dissociating the surfactant/nucleic acid complex with a guanidine isothiocyanate buffer (this is not supposed to work based on the instruction manual of the PAXgene™ Blood RNA Tubes),
(c) extracting mRNA and/or total RNA using an reproducible automated device,
(d) dispersing/distributing a reagent mix for RT-PCR using an automated device,
(e) dispersing/distributing the nucleic acids isolated in step (c) within the dispersed reagent mix of step (d) using an automated device, and,
(f) quantifying RNA by real time PCR, whereby the RT and the PCR are preferably performed in one step, in order to avoid errors.

In this concept of the present invention, the automated device is any device that allows mRNA/RNA/DNA extraction from a guanidine isothiocyanate buffer, in a reproducible manner. The same or another may be used to accurately dispense the reagents and the samples in the reaction tube for the RT-PCR. An 'error' may be a pipetting-, a handling-, a procedural- and/or a calculation error or any error which can be made by a person skilled in the art.

The present invention also refers to a kit for isolating quantifiable *in vivo* RNA from a biological sample comprising:

(a) optionally, a collection tube for biological samples,
(b) a compound inhibiting RNA degradation and/or gene induction,
(c) reagents for automated RNA isolation,
(d) a reagent mix for a simultaneous RT and real-time PCR reaction or separate compounds thereof, allowing the automated dispersion of said mix,
(e) optionally, specific oligonucleotides to perform said RT-PCT reactions, and,
(f) optionally, an instruction manual describing a method for an automated RNA isolation, a method for the automated dispersion of a reagent mix and the dispersion of the isolated nucleic acids for RT- real time PCR, and a method for automated RNA analysis.

In the present examples the inventors are applying the "Lightcycler mRNA hybridisation probes kit" from Roche Diagnostics, Molecular Biochemicals (cat # 3 018 954) to perform the RT-PCR reactions in one step. All reagents needed are included in this kit, except the oligonucleotides (synthesized by Biosource). Nevertheless, real time PCR as described in the present invention can also be performed on Applied Biosystems instruments.
According to the present invention, compound (b) of said kit may be a quaternary amine surfactant such as tetradecyltrimethyl-ammonium oxalate or may be a compound inhibiting RNA degradation and/or gene induction as found in a PAXgene™ Blood RNA Tube, The kit may additionally comprise a buffer such as a guanidine-thiocyanate-containing buffer which can be used in step (d) of the method according to the present invention.
The present invention relates also to a kit for isolating quantifiable *in vivo* RNA from a biological sample comprising:

(a) a PAXgene™ Blood RNA Tube,
(b) a guanidine isothiocyanate buffer,
(c) reagents for automated RNA isolation,
(d) a reagent mix for a simultaneous RT and real-time PCR reaction or separate compounds thereof, allowing the automated dispersion of said mix,
(e) optionally, specific oligonucleotides to perform said RT-PCT reactions, and,
(f) optionally, an instruction manual describing a method for an automated RNA isolation, a method for the automated dispersion of a reagent mix and the dispersion of the isolated nucleic acids for RT- real time PCR, and a method for automated RNA analysis.

The method according to the present invention can also be used for the quantification/ detection of DNA (ds or ss) in biological samples. Therefore, the present invention also relates to a method for the quantification of DNA from a biological sample wherein a method is used as performed for the quantification of RNA according to the present invention, wherein the RT reaction is skipped and wherein the compound of step (a) also protects the DNA from being degraded. As these nucleic acids are more stable than RNA, its stabilization is less important than for RNA.
In addition, the present invention relates to a kit for isolating quantifiable DNA from a biological sample according to the present invention, wherein a reagent mix/ compounds for performing said RT reaction is absent. Situations where exact DNA levels need to be determined in biological samples may be to determine the 'presence' of infection(s)/ contamination(s) in biological samples by unexpected genes, pathogens or parasites; and/or to determine the 'level' of said infection/contamination. For example the method may be used to determine the percentage of transgenic material in a cereal batch.
The present invention also relates to the use of any of the methods or kits as described above, for the monitoring/detection of changes of *in vivo* nucleic acids levels in a biological agent present in a biological sample. With changes is meant presence/absence or decreased/increased levels. With a biological agent is meant all types of agents carrying nucleic acids. With a biological sample is meant a sample carrying a biological agent; the biological sample may be a clinical, agrigultural, environmental, food, forensic sample or any other possible sample.
The method according to present invention may be used for various purposes. E.g. the method can be applied to detect changes in metabolic activity, to identify cellular states, to identify the differentiation of cells, to analyze gene induction, to start expression profiling, to identify cell types by evaluating their transcript content, to study genetic and/ or familial disorders and/or genetic aberrations or to verify genetic identity.
According to the present invention, said biological agent may be chosen for instance from the group consisting of eukaryotic cells, prokaryotic cells, viruses and phages. According to the present invention, the 'eukaryotic cell' may be any eukaryotic cell which is normally present or absent (eg. yeast, fungi, parasites or plant cells) in said sample; 'prokaryotic cells' may be bacteria; 'viruses' may be any RNA or DNA containing virus.
The present invention also relates to the use a method or a kit, according to the present invention, for the

monitoring/detection of changes of *in vivo* nucleic acids of a biological agent in a biological sample, in order to diagnose a certain disease.

The present invention also relates to the use a method or a kit, according present invention, for the monitoring/detection of changes of *in vivo* nucleic acids of a biological agent in a biological sample, in order to screen for a compound for the production of a medicament for curing a disease. Therefore, the invention also relates to a compound identifiable by a method according to present invention.

An example of the disease to be cured or diagnosed is an immuno-related disease. Examples of immuno-related diseases may be rheumatoid arthritis, multiple sclerosis, cancer, immunodeficiencies (AIDS), allergy, graft rejection, GVHD .

The present invention also describes a use of a method or a kit according to the present invention, for the detection/monitoring/screening of a compound, wherein said compound is an immunomodulatory compound which may be chosen from the group consisting of eukaryotic cells, prokaryotic cells, viruses, phages, parasites, drugs (natural extracts, organic molecule, peptide, proteins, nucleic acids), medical treatment, vaccine and transplants. The use of such a method is not limited to detect/monitor/screen a single compound. Synergetic effects of group of substances can also be studied.

The present invention also relates to the use of any of the methods or kits as described above, for the detection/monitoring of epitope specific CTLs or immuno-related transcripts.

The method/kit according to the present invention can also be applied for the monitoring of in-vivo immunological responses after the treatment of patients with a drug/treatment/vaccine susceptible to modify their immune status. According to the invention, the detection of cytokine mRNA (can be extended to chemokine, growth factors, cytotoxic markers, apoptosis markers, or any marker relate to the activation of the immune system known or to be discovered) with the described method in whole blood of patients under therapy or enrolled in clinical trials with an immunomodulator drug or treatment or with a vaccine (therapeutic or prophylactic) may be used to evaluate the efficiency, the safety and/or the eventual by-side effects of the therapy.

The present invention also relates to a method/kit/procedure for the detection of *in vivo* immunological status for the diagnostic/prognostic of diseases affecting the immune system (cancer, auto-immune diseases, allergy, transplant rejection, GVHD, etc.)

According to the invention, the detection of cytokine mRNA (can be extended to chemokine, growth factors, cytotoxic markers, apoptosis markers, or any marker relate to the activation of the immune system known or to be discovered) with the described method in whole blood of patients suffering a disease that affects directly of indirectly their immune system with the aim to dress a diagnosis or prognosis.

The present invention also describes a method to identify an agent capable of modifying the immunological status of a subject via the analysis of epitope specific CTLs comprising the steps of:

(a) applying an immunomodulatory agent(s) into a subject,
(b) sampling whole blood from said subject,
(c) optionally, pulsing blood cells present in the whole blood sample of step (b) with an identical/ similar and/or different immunomodulatory agent as applied in step (a),
(d) collecting pulsed blood cells of step (c) or non-pulsed blood cells of step (b) in a tube comprising a compound inhibiting RNA degradation and/or gene induction, or adding said compound to the pulsed/non-pulsed cells,
(e) forming a precipitate comprising nucleic acids,
(f) separating said precipitate of step (e) from the supernatant,
(g) dissolving said precipitate of step (f) using a buffer, forming a suspension,
(h) isolating nucleic acids from said suspension of step (g) using an automated device,
(i) dispersing/distributing a reagent mix for RT-PCR using an automated device,
(j) dispersing/distributing the nucleic acids isolated in step (h) within the dispersed reagent mix of step (i) using an automated device,
(k) detecting/ monitoring/ analyzing the *in vivo* levels of epitope specific CTLs-related transcripts in the dispersed solution of step (j) in an automated setup, and,
(l) identify agents able to modify the immunological status of said subject,
whereby, in case the agent of step (a) is already present in the subject, step (a) is omitted. According to the present invention the immunomodulatory agent(s) may be present in case of a disease or in the presence of a transplant in said subject. In the present invention the 'epitope specific CTLs-related transcripts' may be transcripts coding for cytokines, cytokine receptors, cytotoxines (like granzyme, perforines, etc.), members of the TNF-related cytokine-receptor superfamily and their ligands (ex: Fas and Fas-ligand) or other cellular receptors.

The present invention also describes a method to identify an agent capable of modifying the immunological status of a subject:

(a) applying an immunomodulatory agent(s) into a subject,
(b) sampling whole blood from said subject,
(c) optionally, pulsing blood cells present in the whole blood sample of step (b) with an identical/ similar and/or different immunomodulatory agent as applied in step (a),
(d) collecting pulsed blood cells of step (c) or non-pulsed blood cells of step (b) in a tube comprising a compound inhibiting RNA degradation and/or gene induction, or adding said compound to the pulsed/non-pulsed cells,
(e) forming a precipitate comprising nucleic acids,
(f) separating said precipitate of step (e) from the supernatant,
(g) dissolving said precipitate of step (f) using a buffer, forming a suspension,
(h) isolating nucleic acids from said suspension of step (g) using an automated device,
(i) dispersing/distributing a reagent mix for RT-PCR using an automated device,
(j) dispersing/distributing the nucleic acids isolated in step (h) within the dispersed reagent mix of step (i) using an automated device,
(k) detecting/ monitoring/ analyzing the *in vivo* levels of immuno-related transcripts in the dispersed solution of step (j) in an automated setup, and,
(l) identify agents able to modify the immunological status of said subject, whereby, in case the agent of step (a) is already present in the subject, step (a) is omitted. In the present invention the 'immuno-related transcripts' may be transcripts coding for e.g. cytokine(s), chemokines(s), growth factors, cytotoxic markers, transcription factors, members of the TNF-related cytokine-receptor superfamily and their ligands, or any markers related to activation of the immune system known or to be discovered. According to the present invention the immunomodulatory agent (s) may be present in case of a disease or in the presence of a transplant in said subject. The subject according to the present invention may be of both human or animal origin.

The present invention also relates to a method for the diagnosis/ prognosis/ monitoring of a clinical status affecting the immune system in a subject comprising the steps of:

(a) sampling whole blood from said subject in a tube comprising a compound inhibiting RNA degradation and/or gene induction, or adding said compound to the blood cells,
(b) forming a precipitate comprising nucleic acids,
(c) separating said precipitate of step (b) from the supernatant,
(d) dissolving said precipitate of step (c) using a buffer, forming a suspension,
(e) isolating nucleic acids from said suspension of step (e) using an automated device,
(f) dispersing/distributing a reagent mix for RT-PCR using an automated device,
(g) dispersing/distributing the nucleic acids isolated in step (e) within the dispersed reagent mix of step (f) using an automated device,
(h) detecting/ monitoring/ analyzing the *in vivo* levels of immuno-related transcripts in the dispersed solution of step (g) in an automated setup, and,
(i) detecting/ monitoring the change in *in vivo* levels of immuno-related transcripts, and,
(j) diagnosing/ prognosing/ monitoring the disease affecting the immune system.

The present invention also provides a method for the diagnosis/ prognosis/ monitoring of a clinical status affecting the immune system in a subject comprising the steps of:

(a) sampling whole blood from said subject,
(b) pulsing blood cells present in the whole blood sample of step (a) with an identical/ similar and/or different immunomodulatory agent as present in the subject,
(c) collecting pulsed blood cells of step (b) in a tube comprising a compound inhibiting RNA degradation and/or gene induction, or adding said compound to the pulsed cells,
(d) forming a precipitate comprising nucleic acids,
(e) separating said precipitate of step (d) from the supernatant,
(f) dissolving said precipitate of step (e) using a buffer, forming a suspension,
(g) isolating nucleic acids from said suspension of step (f) using an automated device,
(h) dispersing/distributing a reagent mix for RT-PCR using an automated device,
(i) dispersing/distributing the nucleic acids isolated in step (g) within the dispersed reagent mix of step (h) using an automated device,
(j) detecting/ monitoring/ analyzing the *in vivo* levels of immuno-related transcripts in the dispersed solution of step (i) in an automated setup, and,
(k) detecting/ monitoring the change in *in vivo* levels of immuno-related transcripts, and,

(I) diagnosing/ prognosing/ monitoring the disease affecting the immune system.
In the present invention 'clinical status' is any change of the physical condition of a subject such as different diseases or presence of transplants.

Unless otherwise defined, all technical and scientific terms used herein have the same meaning as commonly understood by one of ordinary skill in the art to which this invention belongs. Exemplary methods and materials are described below, although methods and materials similar or equivalent to those described herein can be used in the practice or testing of the present invention. All publications and other references mentioned herein are incorporated by reference in their entirety. In case of conflict, the present specification, including definitions, will control. The materials, methods, and examples are illustrative only and not intend to be limiting. Other features and advantages of the invention will be apparent from the following figures, detailed description, and from the claims.
Example 1 is performed by the inventors and makes part of prior art literature (Stordeur et al, J Immunol Methods, 259 (1-2): 55-64, 2002). Nevertheless, it does not include the method as presented by the present invention. As this example describes materials and methods used/followed in the examples which prove the concept of the present invention, this first is included in the present document to make the concept of the present invention clear. Appropriate reference is made to this example. This makes the present document more complete and allows a person skilled in the art to reproduce the invention.

Brief description of the figures and tables

Figure 1.1: **Standard curves for mouse IL-9 reproducibility.** Standard curves were constructed with plasmid dilutions ranging from $10^3$ to $10^7$ copies (for details see text). A typical curve is shown for the TaqMan probe on GeneAmp 5700 (A), for hybridisation probes (B) and the TaqMan probe (C) on Lightcycler.

Figure 1.2. **Typical results for primer titration.** Three different concentrations of each primer may be used: 300, 600 and 900 nM. Each concentration of forward primer is tested in combination with each concentration of reverse primer in a real time PCR reaction starting with $10^5$ copies of purified standard. The curves obtained with the different combinations of primer concentration were nearly similar for TNF-α (top panel), but quite different for IFN-γ (bottom panel). In this latter case, we choose the primer concentrations that give the curve with the higher slope, and the lower Ct value, *i.e.* here 600/600 nM.

Figure 1.3. **Kinetic study for cytokine mRNAs.** PBMC were cultured in medium alone (NS columns) or stimulated with 1 μg/ml PHA, for the indicated time periods. Real time PCR was performed for the different cytokine mRNAs, in four independent experiments. The results were expressed in mRNA copy numbers calculated relative to non-stimulated (NS) cells, after normalisation against β-actin. The mean + SEM (error bars) of the four experiments is represented.

Figure 1.4. **Induction of IL-10 mRNA in human monocytes by IFN-α.** Human monocytes were cultured in medium alone (NS column) or stimulated with 5000 I.U./ml IFN-α for the indicated time periods. Cells were lysed and total RNA was extracted for IL-10 mRNA quantification by real time PCR. Results are expressed in mRNA copy numbers calculated relative to non-stimulated (NS) cells after normalisation against β-actin and represent the mean of three different experiments.

Figure 2.1: **RT-PCR for spontaneous production of IFN-γ and IL-10 mRNAs in peripheral blood.** Total RNA was extracted from whole blood and from PBMC, as stated, from six different healthy volunteers (columns 1 to 6). Whole blood: 0.6 ml of whole blood were mixed with 6 ml of Catrimox-14™, within the minute that follows sample collection. The samples were then centrifuged at 12000 g for 5 min. The resulting nucleic acids pellet was carefully washed with water, and dissolved in 1 ml of Tripure™. RNA extraction was then carried out according to Tripure™ manufacturer's instructions. PBMC: cells were prepared following standard procedures from 15 ml of heparinized venous blood, and lysed in 1 ml of Tripure™ for RNA extraction. RT-PCR for IFN-y, IL-10 and housekeeping gene HPRT were performed for all samples from 1 μg total RNA as described (Stordeur et al., (1995), Pradier et al., (1996)).

Figure 2.2: **Real time PCR for IFN-γ and IL-10 mRNA stability in whole blood.** A sample of citrated venous blood was collected from healthy donors. From this sample, a 100 μl aliquot was mixed with 900 μl of Catrimox-14™, within the minute that follows blood collection, and every hour after during five hours, the blood sample being

simply kept at room temperature between each aliquot taking. The resulting nucleic acids pellet (see legend to Figure 1) was dissolved in 300 µl lysis buffer from the "MagNA Pure LC mRNA Isolation Kit I" (Roche Diagnostics, Molecular Biochemicals). mRNA was extracted using the MagNA Pure LC Instrument (Roche Diagnostics, Molecular Biochemicals) following manufacturer's instructions (final elution volume: 100 µl). Reverse transcription and real time PCR were performed in one step, following the standard procedure described in the "Lightcycler - RNA Master Hybridisation Probes Kit" (Roche Diagnostics, Molecular Biochemicals), starting from 5 µl of the mRNA preparation. Primers and probes sequences, and PCR conditions, are described in Example 1. Results are shown for one representative donor and expressed in mRNA copy numbers normalised against β actin.

Figure 3: **Schematic comparison of the RNA extraction method from whole blood as suggested by PreAnalytiX compared to method as proposed by the present invention.**

Figure 4. **Cytokine blood mRNA ex vivo induction by tetanus toxoid. Tetanus toxoid** (10 µg/ml, Aventis) was added to 500 µl whole blood collected from healthy volunteer vaccinated against tetanus seven years ago. After different time periods at 37°C in a 5% $CO_2$ atmosphere, 1.4 ml of the reagent contained in the PAXgene tube was added. 300 µl of the obtained lysate were used to isolate total mRNA on the MagNA Pure instrument, and RT-PCR was performed as described in the present invention.

Table 1: **Oligonucleotides for real time PCR.**

Table 2: **Oligonucleotides for standard preparation.**

Table 3: **Coefficients of variation (CV) (%).**

Table 4: **Comparison of Qiagen and MagNA Pure LC mRNA extraction methods.**

Modes for carrying out the invention:

*Example 1: Cytokine mRNA quantification by real-time PCR (published in Stordeur et al, J Immunol Methods, 259 (1-2): 55-64, 2002).*

Real time PCR represents a new methodology that accurately quantifies nucleic acids. This has been made possible by the use of fluorogenic probes, which are presented in two forms, namely hydrolysis probes (also called TaqMan probes) and hybridisation probes. The inventors decided to apply this methodology to cytokine mRNA quantification and this led the inventors to the development of a protocol that provides an easy way to develop and perform rapidly real time PCR on a Lightcycler instrument. It was made possible by the use of freely available software that permits a choice of both the hydrolysis probe and the primers. The inventors firstly demonstrated that the reproducibility of the method using hydrolysis probes compares favourably with that obtained with hybridisation probes. The inventors then applied this technique to determine the kinetics of IL-1ra, IL-1β,IL-5, IL-13, TNF-α and IFN-γ induction upon stimulation of human peripheral blood mononuclear cells (PBMC) by phytohaemagglutinin (PHA). Finally, the method was also used successfully to demonstrate that IFN-α induces IL-10 mRNA accumulation in human monocytes.

INTRODUCTION

RT-PCR has become a popular technique with which to obtain insight into the complexity of the immune response. The easy detection of cytokine mRNA transcripts in a limited number of cells where the corresponding protein could barely be measured is probably one of the major advantages of the technique. Nevertheless, RT-PCR suffers from the drawback that it is difficult to quantify accurately the amount of these transcripts. To circumvent this, several (semi)-quantitative RT-PCR techniques have been developed during the last decade, including real time (or kinetic) PCR, which appears nowadays to be the most accurate.
Real time PCR is so called because the amplicon accumulation can be directly monitored during the PCR process, using fluorogenic probes. Two kinds of such probes are currently used, namely the hydrolysis probes (TaqMan probes) that take advantage of the 5'→3' exonuclease activity of *Thermus aquaticus* DNA polymerase (Holland et al., (1991); (Heid et al., (1996); Livak et al., (1995)), and the hybridisation probes that use the fluorescence resonance energy transfer (FRET) phenomenon (Wittwer et al., (1997b); Lay and Wittwer, (1997)). Usually, TaqMan probes are used on a GeneAmp 5700 or an ABI PRISM 7700 apparatus (Applied biosystems), and FRET probes on a Lightcycler apparatus (Roche Diagnostics, Molecular Biochemicals) (Wittwer et al., (1997a)). In this example, the inventors describe a simplified strategy to develop and perform real time PCR for cytokine mRNAs using TaqMan probes on a Lightcycler. First,

the inventors present results obtained on both instruments for reproducibility testing of mouse IL-9 DNA quantification. Thereafter, the inventors applied this protocol to develop real time PCR for human IL-1ra, IL-1β, IL-5, IL-13, TNF-α, IFN-y and β-actin mRNAs, permitting the inventors to monitor the induction of these cytokines upon polyclonal activation of human peripheral blood mononuclear cells (PBMC). Finally, real time PCR for human IL-10 mRNA allowed the inventors to evaluate further the induction of this cytokine by IFNα in monocytes.

MATERIALS AND METHODS

***Cells.*** PBMC were prepared from healthy donors by centrifugation of heparinized venous blood on Lymphoprep (Nycomed, Oslo, Norway Human). Monocytes were isolated from PBMC by a two cycle clumping method as described in a previous study (Stordeur et al., (1995)). Cells were cultured in RPMI 1640 medium + 10 % foetal calf serum (FCS), in a 5 % $CO_2$ atmosphere incubator, at 2 million cells per ml. Phytohaemagglutinin (PHA) and lipopolysaccharide (LPS) were purchased at Sigma-Aldrich (Bornem, Belgium), and IFN-α (IFN-α2b, Intron A®) came from Schering-Plough (Brinny) Co (Innishannon, Ireland).

***Total RNA isolation and reverse transcription.*** Total RNA was isolated using a commercially available reagent (Tripure™, Roche Diagnostics, Molecular Biochemicals, Brussels, Belgium) following the manufacturer's instructions. In some cases, total RNA was treated with 10 units of RQ1 RNase-free DNase (Promega Corporation, Madison, WI) for 30 min, in order to avoid amplification of contaminating genomic DNA (see footnote *** of Table I). After the addition of 500 µl of Tripure™ to inactivate DNase, total RNA was extracted once again. Reverse transcription of mRNA was carried out as follows: 8 µl of water containing 500 ng of total RNA were added to 2 µl of oligo dT primer (0.5 µg/µl), and incubated at 65°C for 10 min. Samples were chilled on ice, and 10 µl of RT mix containing the following components were added: **1)** 4 µl 5x RT buffer (250 mM Tris HCl, pH 8.3, 375 mM KCl, 15 mM $MgCl_2$); **2)** 2 µl deoxynucleotide triphosphate mix (10 mM each); **3)** 0.2 µl bovine serum albumin (1 mg/ml); **4)** 0.6 µl (25 U) human placental ribonuclease inhibitor (RNAguard®, Pharmacia Biotech, Sweden); **5)** 1 µl (200 U) M-MLV reverse transcriptase (Gibco Life Technologies, Scotland, UK); **6)** 0.2 µl $H_2O$; **7)** 2 µl dithiothreitol (100 mM). The samples were then incubated at 37°C for 60 min.

***Primers and probes.*** Except for the mouse IL-9 TaqMan probe that was kindly provided by Applied Biosystems (Applied Biosystems, Foster City, CA), all primers and probes used in this study (sequences listed in Table I) were synthesised at Biosource Europe (Nivelles, Belgium) and designed with the Primer 3 software (Steve Rozen and Helen J. Skaletsky, 1996, 1997, 1998; http://www-genome.wi.mit.edu/cgi-bin/primer/primer3 www.cgi). The default parameters of the program were applied, except for the following: product size 70 to 90 bp, primer size 18 to 27 bp, primer Tm 58 to 62 °C with a max Tm difference of 2.0 °C, product Tm 0 to 85 °C, max self and 3' self complementarity for primers = 6.00, max poly-X = 3, primer and Hyb Oligo penalty (penalty weights for primer pairs) = 2.0, Hyb Oligo Tm 68 to 72 °C, max self and 3' self complementarity for Hyb Oligo = 6.00, max poly-X = 3. Within the proposed oligonucleotides, primers and probe were selected following these criteria: (in order of importance) 1) intron spanning if possible, 2) no G in 5' for the probe, 3) no more than two G or C within the five last nucleotides in 3' for the primers, 4) more C then G in the probe.

***PCR on the GeneAmp 5700 for mouse IL-9 DNA.*** The PCR reaction was carried out in a 50 µl final volume containing: **1)** $H_2O$ up to 50 µl; **2)** 25 µl Universal PCR Master Mix (Applied Biosystems); **3)** 1 µl of 6 pmoles/µl for each forward and reverse primer (final concentration 300 nM); **4)** 1µl of 4 pmoles/µl TaqMan probe (final concentration 200 nM); **5)** 5 µl of plasmid dilution. After an initial denaturation step at 94°C for 10 min, temperature cycling was initiated. Each cycle consisted of 94°C for 15 s and 60°C for 60 s, the fluorescence being read at the end of this second step. 45 cycles were performed, in total.

***PCR on the Lightcycler with hybridisation probes (mouse IL-9 DNA).*** The PCR reaction was carried out in a 20 µl final volume containing: **1)** $H_2O$ up to 20 µl; **2)** 2 µl DNA Master Hybridisation Probes 10x (DNA Master Hybridisation Probes Kit - Roche Diagnostics, Molecular Biochemicals); **3)** 5 µl 25 mM $MgCl_2$; **4)** 1 µl of 6 pmoles/µl forward and 3 µl of 6 pmoles/µl reverse primers (final concentration 300 and 900 nM, respectively); **5)** 1µl of 4 pmoles/µl of each of both hybridisation probes (final concentration 200 nM); **6)** 0.3 µl anti-Taq DNA polymerase antibody (Platinums *Taq* antibody, Gibco Life Technologies); **7)** 1 µl of plasmid dilution. After an initial denaturation step at 95°C for 30 s, temperature cycling was initiated. Each cycle consisted of denaturation at 95°C for 0 (zero) second, hybridisation at 59°C for 10 s, and elongation at 72°C for 10 s. The fluorescent signal was acquired at the end of the hybridisation step (F2/F1 channels, fluorimeter gains regulated on 1 for F1, 15 for F2 and 30 for F3, with color compensation). 45 cycles were performed, in total.

***PCR on the Lightcycler with TaqMan probes.*** The PCR reaction was carried out in a 20 µl final volume containing: **1)** $H_2O$ up to 20 µl; **2)** 2 µl DNA Master Hybridisation Probes 10x (DNA Master Hybridisation Probes Kit - Roche Diagnostics, Molecular Biochemicals); **3)** 5 µl 25 mM $MgCl_2$; **4)** 1, 2 or 3 µl of 6 pmoles/µl forward and reverse primers (final concentration 300, 600 or 900 nM, see Table I); **5)** 1µl of 4 pmoles/µl TaqMan probe (final concentration 200 nM); **6)** 0.3 µl anti-Taq DNA polymerase antibody (Platinums *Taq* antibody, Gibco Life Technologies); **7)** 1 µl cDNA

or standard dilution. After an initial denaturation step at 95°C for 30 s, temperature cycling was initiated. Each cycle consisted of 95°C for 0 (zero) second and 60°C for 20 s, the fluorescence being read at the end of this second step (F1/F2 channels, fluorimeter gains regulated on 8 for F1, 2 for F2 and 4 for F3, without color compensation). 45 cycles were performed, in total.

***Standard curves and results expression.*** mRNA levels were expressed either in absolute copy numbers or in relative copy numbers normalised against β-actin mRNA. This was achieved by constructing, for each PCR run, a standard curve from serial dilutions of a purified DNA. This latter consisted of a PCR product that included the quantified amplicon, and that was prepared by "classical" PCR from cDNA positive for the concerned target mRNA. These PCR products used as standards were purified from agarose gel following standard procedures, at the end of which the copy number was calculated as described (Overbergh et al., (1999)). Detailed information concerning these standards is given in Table II. For human IL-5 and mouse IL-9, the serial dilutions were made from a purified plasmid (mouse IL-9 plasmid was kindly provided by Dr Jean-Christophe Renauld from the Ludwig Institute, Brussels, Belgium, and human IL-5 plasmid was purchased from the American Type Culture Collection, Manassas, VA).
The mRNA copy numbers were calculated for each sample from the standard curve by the instrument software, using the Ct value ("Arithmetic Fit point analysis" for the Lightcycler). Results were expressed in absolute copy numbers, or in copy numbers calculated relative to unstimulated cells, after normalisation against β-actin mRNA, as follows.

For each sample, a corrected cytokine mRNA copy number (CN) was firstly calculated:

$$\text{Corrected cytokine mRNA CN} = (\text{cytokine mRNA CN}/\beta\text{-actin mRNA CN}) * \beta\text{-actin mRNA CN of unstimulated cells}$$

Then the relative copy number was obtained from the formula:

$$\text{Relative CN (\%)} = (\text{corrected cytokine mRNA CN}/\text{corrected cytokine mRNA CN of unstimulated cells}) * 100$$

This normalisation against the house keeping gene is possible only if both PCR (cytokine + house keeping genes) present the same efficiency. This latter has been calculated for each PCR run from the slope of the standard curve:

$$\text{Efficiency (E)} = 10^{(-1/slope)}$$

and found to be nearly similar for all PCR reactions presented in this study. If that had not been the case, the inventors could have expressed their results in ΔΔCt instead of relative copy numbers. In this case, for each sample, two "ΔCt" can be calculated:

$$\Delta Ct\ \text{cytokine} = Ct\ \text{cytokine of the sample} - Ct\ \text{cytokine of unstimulated cells}$$

$$\Delta Ct\ \beta\text{-actin} = Ct\ \beta\text{-actin of the sample} - Ct\ \beta\text{-actin of unstimulated cells}$$

and used for the determination of the ΔΔCt: (for each sample)

$$\Delta\Delta Ct = [1\ (E_{cy}/E_{act})]^{-(\Delta Ct\ \text{cytokine} - \Delta Ct\ \beta\text{-actin})} * 100$$

where $E_{cy}$ = efficiency of cytokine PCR and $E_{act}$ = efficiency of β-actin PCR.

RESULTS.

***Use of TaqMan chemistry on the Lightcycler: reproducibility of mouse IL-9 DNA quantification.*** The inventors transposed to the Lightcycler the protocol recommended by Applied Biosystems for TaqMan probe use on a GeneAmp 5700. To confirm that this protocol gave the same results on both types of apparatus, the inventors compared the reproducibility of mouse IL-9 DNA quantification on the two instruments, for the two kinds of probes (except for hybridisation probes that could not be used on GeneAmp 5700). This was carried out with three dilutions of a plasmid that contained the coding sequence of the cytokine. The PCR were performed for each plasmid dilution twenty times in one experiment (intra-run coefficient of variation (CV)), and once in twenty different experiments (inter-run CV). The

copy numbers were calculated for each dilution from a standard curve constructed with serial dilutions of the plasmid (Figure 1.1). The CV of the copy numbers was calculated for each dilution (Table III-A). The inventors found (1) that the TaqMan probe on the Lightcycler offered a better reproducibility than hybridisation probes, especially for low copy numbers in inter-run assays, and (2) that the intra-run reproducibility was similar and satisfactory for the two instruments, while the Lightcycler globally presented a better but unsatisfactory inter-run reproducibility. On this basis, the inventors decided to express their results in copy numbers calculated relative to unstimulated cells, after normalisation against β-actin.

***Quantification of human IL-1ra, IL-1β, IL-5, IL-13, TNF-α and IFN-γ mRNAs.*** The strategy proposed in example 1 to develop real time PCR is simple and includes the following steps: (1) primers and probe choice with the primer 3 software; (2) preparation of the standard by "classical" PCR; (3) primer titration. The inventors successfully applied it for different cytokine mRNAs and effectively found that the only necessary adaptation was the primer titration (Figure 1.2). This technique was then used in a kinetic study of human PBMC stimulated with PHA. The inventors quantified the mRNA levels of IL-1ra, IL-1β, IL-5, IL-13, TNF-α, IFN-y and β-actin at different times of culture. Figure 1.3 illustrates the capacity of this system to quantify cytokine mRNAs. A similar pattern, *i.e.* a rapid but transient induction, peaking around four to eight hours after PHA addition, was seen for the different cytokines.

***Human IL-10 transcript quantification: monocytic IL-10 mRNA induction by IFN-α.*** The inventors developed IL-10 mRNA quantification, in order to study the capacity of IFN-α to enhance IL-10 mRNA levels, which was demonstrated in previous studies (Schandené et al., (1996); Aman et al., (1996)). First, the inventors evaluated the reproducibility of the method for IL-10 mRNA, using a cDNA from LPS-stimulated PBMC. The coefficient of variation obtained was satisfactory as shown in Table III-B. Data presented in Figure 1.4 confirmed that IFN-α induces a clear and transient induction of IL-10 mRNA in purified monocytes.

## DISCUSSION

The development of real time PCR to quantify a (c)DNA copy number represents a major step forward in PCR technology. It is now routinely applied in cancer for the evaluation of minimal residual disease (Nakao et al., (2000); Verhagen et al., (2000)), as well as for the detection of bacterial and viral infections (Lyons et al., (2000); Yun et al., (2000); Josefsson et al., (2000)). Apart from these applications, real time PCR has been used little to date for cytokine mRNA quantification (Brink et al., (2000); Overbergh et al., (1999); Blaschke et al., (2000)). However, as demonstrated by Wang and Brown (1999), real time PCR gives similar results than the RNase protection assay, a technique widely used to quantify cytokine mRNAs (these authors demonstrated a strong correlation between the two techniques, with a higher sensitivity for the kinetic PCR). In the same vein, a recent study showed that real time PCR for IL-16 mRNA quantification gave the same results as competitive conventional RT-PCR (Blaschke et al., (2000)). These observations led the inventors to develop real time PCR for cytokine mRNA quantification using TaqMan chemistry on a Lightcycler. The use of FRET technology requires the simultaneous choice of four different oligonucleotides (two primers and two probes) and no available software existed for such a design, whereas the selection of TaqMan probes and primers was possible through freeware on the Net. Moreover, the successful use of hydrolysis probes on a Lightcycler has already been described (Kreuzer et al., (1999)).

The protocol for kinetic PCR with TaqMan probes on the GeneAmp 5700 was readily transposed. In order to optimise the technique, the inventors investigated different conditions and found that a unique protocol can be used for different target mRNAs. This was exactly as suggested by Applied Biosystems for the GeneAmp 5700, the only adjustment required for a new mRNA target design being the titration of primer concentrations (300, 600 or 900 nM of each primer). The inventors evaluated the reproducibility of the protocol and found satisfactory intra-run coefficients of variation for both apparatus, and a better reproducibility for TaqMan probes compared to hybridisation probes, especially for inter-run assays. These results validated the use of TaqMan probes on a Lightcycler. Nevertheless, because of the inter-run coefficients of variation, the inventors expressed their results in copy numbers calculated relative to a reference sample, after normalisation against a house keeping gene. As far as the intra-run coefficients of variation are concerned, similar results have been obtained in previous studies (Overbergh et al., (1999), Bolufer et al., (2000), Gerard et al., (1998)).

The methodology described in the present example was easily and successfully applied to the quantification of several cytokine genes. It was first used to determine the magnitude and the kinetics of induction of cytokine mRNAs upon polyclonal activation of PBMC. Then, the inventors took advantage of the technique to specify the effects of IFN-α on IL-10 mRNA accumulation in human monocytes. Indeed, it has been previously suggested that IL-10 might mediate some of the anti-inflammatory properties of type I interferons (Schandené et al., (1996); Aman et al., (1996)). However, the influence of IFN-α on the production of IL-10 by monocytes is controversial and seems to depend on the activation system considered (Pawelec et al., (1999); Hermann et al., (1998)). The high sensitivity of the real-time PCR method permitted the inventors to demonstrate unambiguously that IFN-α triggers IL-10 mRNA induction in monocytes in the absence of any other stimulus. Apart from the data presented in this paper, the inventors also successfully used the

same approach to demonstrate an impaired synthesis of IL-12 (p35) mRNA by neonatal dendritic cells (Goriely et al., (2001)), providing thereby a molecular basis for the deficient Th1-type immune responses in the newborn.
In conclusion, the inventors provide in the present example a simple strategy to perform and develop quantitative real time PCR for cytokine mRNA quantification. A unique protocol is used for different target mRNAs, the only adjustment being the primer titration, so that real time PCR for a new target mRNA is rapidly developed. This is, in part, due to the use of the primer 3 software that permits the simultaneous choice of the probe and the primers, leading to successful oligonucleotide design. In this way it is possible to avoid the use of a fluorescent dye (e.g. SYBR Green or ethidium bromide), which is less sensitive and less specific than the probe. The inventors suggest that this technique has many advantages for researchers wishing to quantify cytokine mRNAs, and could provide powerful insights into the complexities of the cytokine network.

*Example 2: Analysis of Spontaneous Cytokine mRNA Production in Peripheral Blood*

The quantification of the cytokine mRNAs synthesized by peripheral blood cells should make it possible to estimate a "peripheral immune statute". However, an accurate quantification can only be performed from a fresh whole blood sample in which mRNA is protected against nuclease digestion, and where gene transcription is inhibited. As discussed in this note, this has been made possible by the use of surfactant reagents such as tetradecyltrimethylammonium oxalate. RT-PCR for the quantification of IL-10 and IFN-γ mRNAs spontaneously produced in peripheral blood was performed. The results showed pronounced higher IFN-y transcript levels in whole blood compared to peripheral blood mononuclear cells (PBMC) from the same individuals, while no significant difference was observed for IL-10 mRNA. The higher amounts of IFN-y mRNA observed in blood can be attributed at least to mRNA degradation. Using a real time PCR technique, it could indeed be demonstrated that blood IFN-γ mRNA is rapidly degraded in vitro, the t ½ being worth approximately one hour at room temperature.

Härtel et al. recently analysed the influence of cell purification procedure on spontaneous cytokine mRNA production in peripheral blood (Hartel et al., 2001). They showed that freshly isolated peripheral blood mononuclear cells (PBMC) expressed higher levels of IL-2, IL-4 and TNF-α mRNA than freshly collected whole blood from the same individual, while no difference in IFN-y mRNA level was observed. The inventors performed such a comparison for IFN-y in six different individuals, and found different results. The inventors indeed observed a strong expression of IFN-y mRNA in whole blood of all donors, which is clearly decreased in PBMC (Figure 2.1). This difference between the results obtained by the inventors and those of Härtel et al, despite the fact that these latter used a quantitative real time PCR technique, could be related to the procedure used to isolate total RNA from whole blood. Härtel et al. used heparinized blood that was hemolyzed within two hours by isotonic ammonium chloride treatment. The inventors used tetradecyltrimethylammonium oxalate, a cationic surfactant reagent called Catrimox-14™ (Qiagen, Westburg, Leusden, The Netherlands) that is directly mixed with the blood, avoiding the use of anticoagulants (Dahle and Macfarlane, (1993); Schmidt et al., (1995)). Moreover, this reagent induces nucleic acids precipitation and nuclease inhibition, in the minute that follows sample collection. This provides a total RNA preparation that is probably the nearest of *in vivo* mRNA status. This is especially important for cytokine mRNA, which are made sensitive to endogenous nucleases by their AU-rich sequences located in their 3' untranslated region. Using a real time PCR technique, the inventors indeed observed that peripheral blood IFN-γ mRNA is spontaneously and rapidly degraded, the levels being decreased by roughly 50 % already one hour after blood collection. However, this phenomenon is not necessary true for all the cytokines, as the inventors found that IL-10 mRNA level is stable for at least the five hours that follow blood sampling (Figure 2.2). Moreover, the inventors did not find significant differences in whole blood IL-10 mRNA levels, compared to those of PBMC (Figure 2.1).

The nucleic acids pellet obtained after Catrimox-14™ lysis (see legend to Figure 2.1) can be dissolved in the guanidium/thiocyanate solution described by Chomczynski and Sacchi (1987), as well as in its commercially available version, such as Tripure™ Roche Diagnostics, Molecular Biochemicals, Brussels, Belgium), making the use of this surfactant particularly easy. This means that, except for the first step with Catrimox-14™, the RNA isolation procedure is the same for whole blood and cells. Alternatively, PAXgene™ Blood RNA Tubes (Qiagen, Westburg, Leusden, The Netherlands) could be used in the place of Catrimox-14™. In this case, the resulting pellet can be dissolved in the lysis buffer of the "MagNA Pure LC mRNA Isolation Kit I", as described for Catrimox-14™ in legend to Figure 2.2. The characterisation of spontaneous IL-10 mRNA production by human mononuclear blood cells (Stordeur et al., (1995)), and the monitoring of *in vivo* tissue factor mRNA induction by OKT3 monoclonal antibody (Pradier et al., (1996)), represent two examples
where Catrimox-14 was successfully used. The inventors also observed a strong IL-2 mRNA induction after addition of ionophore A23187 + phorbol myristate acetate to whole blood (not shown), suggesting its use for *in vitro* studies on whole blood.

The observations made by the inventors stress the importance to perform RT-PCR from whole blood lysed as fast as possible, in order to accurately quantify peripheral blood cytokine mRNA. For this purpose, the use of reagents

such as Catrimox-14 or the additive contained in the PAXgene™ Blood RNA Tubes, together with real time RT-PCR, probably represents to-date the best procedure. By doing so, the study of the natural status of peripheral blood cells would be possible without the use of *in vitro* strong stimuli such as ionomycin or phytohaemagglutinin.

*Example 3: Comparison between the PAXgene™ Blood RNA System and proposed method according to the present invention.*

With the 'PAXgene™ Blood RNA System' is meant the combination of the PAXgene™ Blood RNA Tube' with the 'PAXgene™ Blood RNA Kit'. With the 'Qiagen Method', it is meant 'PAXgene™ Blood RNA Kit'.

Based on the experimental evidence of example 2 of the present invention, the inventors propose a new procedure to isolate mRNA from whole blood which allows to determine *in vivo* transcript levels using an easy and reproducible method. The PAXgene™ blood RNA System and the method according to present invention are schematically compared in Figure 3.

Material and methods:

All experiments were performed from peripheral venous blood directly collected in PAXgene™ Blood RNA Tubes as recommended by the PAXgene™ Blood RNA System (Qiagen) (*i.e.* 2.5 ml of blood were vacuum collected within the tube that contains 6.9 ml of an unknown reagent). After lysis completion, the content of the tube was transferred in two other tubes : 4.7 ml were used for PAXgene blood RNA kit, and 0.4 ml for MagNA Pure extraction. The remaining of the lysate was discarded. These two tubes were centrifuged at 2,000 g for 10 min and the supernatant discarded. The nucleic acid pellet was then:

***a) PAXgene™ Blood RNA Tube + PAXgene™ Blood RNA Kit-*** ... washed in water before being dissolved in BR1 buffer for total RNA extraction, as recommended in the corresponding instruction manual. The procedure of the PAXgene™ Blood RNA System is as follows: Blood samples (2.5 ml) are collected in PAXgene Blood RNA Tubes, and may be stored or transported at room temperature if desired. RNA isolation begins with a centrifugation step to pellet nucleic acids in the PAXgene Blood RNA Tube. The pellet is washed, and Proteinase K is added to bring about protein digestion. Alcohol is added to adjust binding conditions, and the sample is applied to a spin column as provided by the PAXgene™ Blood RNA Kit. During a brief centrifugation, RNA is selectively bound to the silica-gel membrane as provided by the PAXgene™ Blood RNA Kit as contaminants pass through. Following washing steps, RNA is eluted in an optimized buffer. Reverse transcription and real time PCR for IFN-γ and β-actin mRNAs were conducted as described in example 1 "Cytokine mRNA Quantification by Real Time PCR" (Stordeur et al, J Immunol Methods, 259 (1-2): 55-64, 2002).

- b) ***PAXgene™ Blood RNA Tube + + MagNA Pure LC mRNA Isolation Kit I -*** ... dissolved in 300 µl lysis buffer from the MagNA Pure mRNA Isolation Kit. Extraction and purification of mRNA in a final elution volume of 100 µl were then performed on the MagNA Pure LC Instrument following the instructions from Roche Diagnostics, Molecular Biochemicals.
Reverse transcription and real time PCR were conducted in one step, following the standard procedure described in the "Lightcycler - RNA Master Hybridisation Probes Kit" (Roche Diagnostics, Molecular Biochemicals), starting from 5 µl of the mRNA preparation.

Results:

The inventors performed the comparison of the extraction method recommended by Qiagen in combination with the PAXgene™ Blood RNA Tubes (PAXgene™ Blood RNA System), with the MagNA Pure LC Instrument extraction method also in combination with the PAXgene™ Blood RNA Tubes. In both methods the use of the PAXgene™ blood RNA Tubes allows to stabilize RNA from blood cells. The results are listed in Table 4.1 and 4.2. The results of this experiment show a better reproducibility for the MagNA Pure LC Technique (coefficients of variation for IFN-y mRNA copy numbers corrected against β-actin are 26 versus 16 % for Qiagen versus MagNA Pure LC, respectively).

It is interesting to note that MagNA Pure extraction was performed from a starting blood volume lower than that used with the Qiagen method (0.11 ml for MagNA Pure versus 1.25 ml for Qiagen). If the Qiagen method had been performed with such small volume, it would be impossible to measure the RNA concentration, even to perform the reverse transcription. This stresses another advantage of the technique described in the present invention : the possibility to quantify mRNA in a very small volume of blood (about 100 µl).

Conclusion:

Example 3 illustrates the possibility to use the PAXgene™ Blood RNA Tubes in combination with the MagNA Pure LC mRNA Isolation Kit I, or more precisely, the possibility to dissolve the precipitate from the PAXgene™ Blood RNA Tube in the lysis buffer contained in that kit, this lysis buffer necessarily having to be used with the other components of the kit.
The present inventors prove in this example that in contrast to other combinations, only the combination as described in the present invention, leads to correct/real *in vivo* transcript quantification.

*Example 4: Ex vivo monitoring of immune response against tetanus toxoid.*

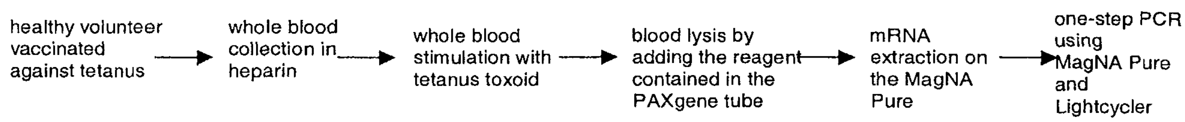


In example 4, blood is stimulated ex vivo with an antigen (i.e. tetanus toxoid) against which the blood donor is supposed to be immunised (because vaccinated seven years ago). RT-PCR is performed according to the method. Cytokine mRNA is measured as a read out of the ability of the volunteer's immune system to react against the antigen. The IL-2, IL-4, IL-13 and IFN-γ mRNAs are preferentially analysed, but all potentially reactive proteins can be analysed via the quantification of their corresponding mRNA. Results of example 4 is shown in figure 4.
Generally the strategy followed in this example can be schematically represented as follows:

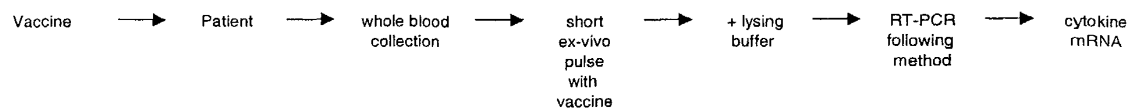


Example of possible application: Cancer immunotherapy Since some years, basic strategies on cancer immunotherapy evolved in the way of the vaccination. In fact, the progresses in genetic and in immunology have allowed identifying a number growing tumor antigens that are expressed to the surface of tumor cells. These antigens are presented to the surface of tumor cells under the form of peptides associated to the major histocompatibility complex (HLA). Example of antigens that might be considered as tumor antigens are described by Fong and Engleman (Annu. Rev. Immunol. 2000. 18:245-273). The principle of the anti-cancer vaccination consists to present these antigens to the system immune of the patient following the most immunogenic way immunogenic. That goes from the injection of the antigen or corresponding peptides in the presence of additives to the presentation of the peptide on autologous antigen presenting cells (dendritic cells, for example). Although the ultimate goal of vaccination anti-cancer vaccination remains the regression of the tumor, the determination of the efficiency of anti-cancer vaccination remains difficult especially in the case of patients in advanced phase of the disease that can profit only from a limited window of treatment. It is the reason why the anti-cancer vaccination could especially be interesting as adjuvant therapy or in the framework of the prevention. It is therefore extremely important to develop sensitive and precise monitoring techniques to evaluate the immunological effects of the experimental anti-cancer vaccination in order to specify the method of administration of these vaccines and discover the implied biological mechanisms that will be able to help better to define the futures therapeutic protocols. The difficulty to measure the immunological efficiency of these vaccines resides essentially in the absence of assays sufficiently sensitive to detect a cellular immune response *in vivo.* Until now, the used techniques implied the intensive *in vitro* culture of the PBMC of patients on of long periods times in the presence of antigen and of co-stimulating susceptible to induce a modification of the original functional characteristics of lymphocytes. Thus, the analyses of the anergic states or tolerant states of the lymphocyte precursors directed against the tumor antigens is extremely difficult being given the reversible nature of their functional state after their extended in-vitro incubation in the presence of antigen. On the other side, techniques based on tetramers of MHC-peptides complexes that are used for the detection of low frequencies of epitope-specific-CTL precursors lack usually sensitiveness for the detection of tumor-specific lymphocytes. In addition these techniques do not give any information on the functional reactivity of these lymphocytes

Only techniques that are sensitive enough to be able to detect an original functional reactivity of the lymphocytes to a given antigen, for example after a very short stimulation *in vitro* with antigen will allow a real evaluation of the efficiency of anti-cancer vaccination protocols.

It has been shown recently (Kammula, U. S., Marincola, F. M., and Rosenberg, S. A. (2000) Real-time quantitative polymerase chain reaction assessment of immune reactivity in melanoma patients after tumor peptide vaccination. J. Natl. Cancer Inst. 92: 1336-44) that the detection of cytokine mRNA associated to a short in-vitro stimulation (2 hours) of PBMC were able to detect epitope-specifiq CTLs in the PBMC's of patients undergoing vaccination with a tumor antigen. Nevertheless, according to present inventors this short *ex vivo* pulse is not essential.

*Example 5: Detection of the activation of the immune system of the recipient by the histocompatibility antigens of the donor.*

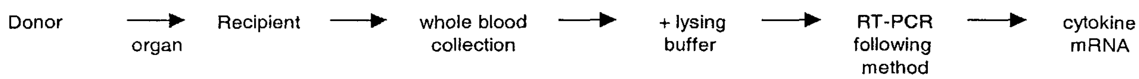


In example 5, an organ (ex. liver, kidney, bone marrow, etc.) from a donor is transplanted to a recipient. Whole blood the recipient is collected in a tube comprising a compound inhibiting RNA degradation and/or gene induction according to present invention. RT-PCR is performed according to the method. Cytokine mRNA is measured as a read out of the activation of the immune system of the recipient by the histocompatibility antigens of the donor.

*Example 6: Detection of the reactivity of the immune* system *of the recipient to the histocompatibility antigens of the donor.*

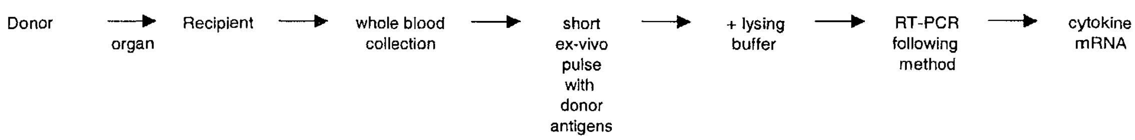


In example 6, an organ (ex. liver, kidney, bone marrow,...) from a donor is transplanted to a recipient. Whole blood of the recipient is collected on a tube and incubated *ex-vivo* with the histocompatibility antigens of the donor. A compound inhibiting RNA degradation and/or gene induction according to present invention is added to the blood. RT-PCR is performed according to the method. Cytokine mRNA is measured as a read out of the response of the immune system of the recipient by the histocompatibility antigens of the donor.

<u>Example of application</u>: monitoring of rejection after organ transplantation

The monitoring of rejections of transplants is essentially based on the detection of markers measured in the urine or the blood of patients (blood urea nitrogen-BIN- or creatinine in the case of kidney transplants) or at the time of the analyses of biopsies of the grafted organ.
These indicators are however only detected when the rejection mechanism is already well advanced. In fact, transplant rejection is the result of an immunological mechanism that precedes the deterioration of the grafted organ. The detection of these immunological mechanisms before the grafted organ is damaged would allow to reduce in a considerable manner the loss of the grafted organ by adapting more earlier the immunosuppressive treatments. On the other side, it is also recognized that of sub-clinical episodes of rejections (with no induction of clinical signs) occur themselves frequently after transplantation. These episodes sub-clinical rejection episodes could be the cause of chronic rejections. Several authors have investigate the detection of precocious immunologiques markers of organ rejection and particularly the detection in the circulation of recipient alloreactive T-lymphocytes directed against the allo-antigens of the donor. Methods include essentially the association of mixed cultures with the consecutive measurement of the proliferation of the lymphocytes of the receiver or the measurement of the production of cytokines by different methods (ELISA, ELISPOT, flow cytometry, etc.). More recently, other authors have looked on the characterization of lymphocytes activation markers patterns susceptible to underline precociously the triggering of a rejection mechanism. The detection of mRNA of genes expressed by the cytotoxic activated T-lymphocytes T activated (granzyme B, perforine, different cytokines) by sensitive methods of quantitative PCR were showed to be excellent tools to measure the triggering of a rejection. For this purpose, according to present invention, messengers coding for different kinds of cytokines may be studied, preferential targets may be IL-2, IFN-gamma, IL-4, IL-5, Granzyme, perforine and FasFas-ligand.

Its is to be understood that while the invention has been described in conjunction with the detailed description thereof, the foregoing description is intended to illustrate and not limit the scope of the invention, which is defined by the scope of the appended claims. All of the references cited in the description are incorporated by reference. Other aspects, advantages, and modifications are within the scope of the following claims.

References

References example 1

Aman, M.J., Tretter, T., Eisenbeis, I., Bug, G., Decker, T., Aulitzky, W.E., Tilg, H., Huber, C. and Peschel, C. (1996) Interferon-alpha stimulates production of interleukin-10 in activated CD4(+) T cells and monocytes. Blood 87, 4731.

Blaschke, V., Reich, K., Blaschke, S., Zipprich, S. and Neumann, C. (2000) Rapid quantitation of proinflammatory and chemoattractant cytokine expression in small tissue samples and monocyte-derived dendritic cells: validation of a new real-time RT-PCR technology. J Immunol Methods 246, 79.

Bolufer, P, Sanz, GF, Barragan, E, Sanz, MA, Cervera, J, Lerma, E, Senent, L, Moreno, I and Planelles, MD. (2000) Rapid quantitative detection of BCR-ABL transcripts in chronic myeloid leukemia patients by real-time reverse transcriptase polymerase-chain reaction using fluorescently labeled probes. Haematologica 85 (12), 1248

Brink, N., Szamel, M., Young, A.R., Wittern, K.P. and Bergemann, J. (2000) Comparative quantification of IL-1beta, IL-10, IL-10r, TNFalpha and IL-7 mRNA levels in UV-irradiated human skin in vivo. Inflamm.Res. 49(6.),290.

Gerard, C.J., Olsson, K., Ramanathan, R., Reading, C. and Hanania, E.G. (1998) Improved quantitation of minimal residual disease in multiple myeloma using real-time polymerase chain reaction and plasmid-DNA complementarity determining region III standards. Cancer Res 58 (17), 3957

Goriely, S., Vincart, B., Stordeur, P., Vekemans, J., Willems, F., Goldman, M. and De Wit, D. (2001) Deficient IL-12(p35) Gene Expression by Dendritic Cells Derived from Neonatal Monocytes. J Immunol 166, 2141.

Heid, C.A., Stevens, J., Livak, K.J. and Williams, P.M. (1996) Real time quantitative PCR. Genome Res 6, 986.

Hermann, P., Rubio, M., Nakajima, T., Delespesse, G. and Sarfati, M. (1998) IFN-alpha priming of human monocytes differentially regulates gram-positive and gram-negative bacteria-induced IL-10 release and selectively enhances IL-12p70, CD80, and MHC class I expression. J Immunol 161, 2011.

Holland, P.M., Abramson, R.D., Watson, R. and Gelfand, D.H. (1991) Detection of specific polymerase chain reaction product by utilizing the 5'----3' exonuclease activity of Thermus aquaticus DNA polymerase. Proc Natl Acad Sci U S A 88, 7276.

Josefsson, A.M., Magnusson, P.K., Ylitalo, N., Sorensen, P., Qwarforth-Tubbin, P., Andersen, P.K., Melbye, M., Adami, H.O. and Gyllensten, U.B. (2000) Viral load of human papilloma virus 16 as a determinant for development of cervical carcinoma in situ: a nested case-control study. Lancet 355, 2189.

Kreuzer, K.A., Lass, U., Bohn, A., Landt, O. and Schmidt, C.A. (1999) LightCycler technology for the quantitation of bcr/abl fusion transcripts. Cancer Res 59, 3171.

Lay, M.J. and Wittwer, C.T. (1997) Real-time fluorescence genotyping of factor V Leiden during rapid-cycle PCR. Clin Chem 43, 2262.

Livak, K.J., Flood, S.J., Marmaro, J., Giusti, W. and Deetz, K. (1995) Oligonucleotides with fluorescent dyes at opposite ends provide a quenched probe system useful for detecting PCR product and nucleic acid hybridization. PCR Methods Appl 4, 357.

Lyons, S.R., Griffen, A.L. and Leys, E.J. (2000) Quantitative real-time PCR for Porphyromonas gingivalis and total bacteria. J Clin Microbiol 38, 2362.

Nakao, M., Janssen, J.W., Flohr, T. and Bartram, C.R. (2000) Rapid and reliable quantification of minimal residual disease in acute lymphoblastic leukemia using rearranged immunoglobulin and T-cell receptor loci by LightCycler technology.

Cancer Res 60(12),3281.

Overbergh, L., Valckx, D., Waer, M. and Mathieu, C. (1999) Quantification of murine cytokine mRNAs using real time quantitative reverse transcriptase PCR. Cytokine 11, 305.

Pawelec, G., Schlotz, E. and Rehbein, A. (1999) IFN-alpha regulates IL 10 production by CML cells in vitro. Cancer Immunol Immunother 48, 430.

Schandené, L., Delprete, G.F., Cogan, E., Stordeur, P., Crusiaux, A., Kennes, B., Romagnani, S. and Goldman, M. (1996) Recombinant interferon-alpha selectively inhibits the production interleukin-5 by human CD4(+) T cells. J.Clin.Invest. 97, 309.

Stordeur, P., Schandené, L., Durez, P., Gerard, C., Goldman, M. and Velu, T. (1995) Spontaneous and cycloheximide-induced interleukin-10 mRNA expression in human mononuclear cells. Mol.Immunol. 32, 233.

Verhagen, O.J., Willemse, M.J., Breunis, W.B., Wijkhuijs, A.J., Jacobs, D.C., Joosten, S.A., van Wering, E.R., van Dongen, J.J. and van der Schoot, C.E. (2000) Application of germline IGH probes in realtime quantitative PCR for the detection of minimal residual disease in acute lymphoblastic leukemia. Leukemia 14, 1426.

Wang, T. and Brown, M.J. (1999) mRNA quantification by real time TaqMan polymerase chain reaction: validation and comparison with RNase protection. Anal.Biochem 269, 198.

Wittwer, C.T., Herrmann, M.G., Moss, A.A. and Rasmussen, R.P. (1997b) Continuous fluorescence monitoring of rapid cycle DNA amplification. Biotechniques 22, 130.

Wittwer, C.T., Ririe, K.M., Andrew, R.V., David, D.A., Gundry, R.A. and Balis, U.J. (1997a) The LightCycler: a microvolume multisample fluorimeter with rapid temperature control. Biotechniques 22, 176.

Yun, Z., Lewensohn-Fuchs, I., Ljungman, P. and Vahlne, A. (2000) Real-time monitoring of cytomegalovirus infections after stem cell transplantation using the TaqMan polymerase chain reaction assays. Transplantation 69, 1733.

References example 2

Chomczynski, P. and Sacchi, N. (1987) Single-step method of RNA isolation by acid guanidinium thiocyanate-phenol-chloroform extraction. Analyt Biochem 162, 156.

Dahle, C.E. and Macfarlane, D.E. (1993) Isolation of RNA from cells in culture using Catrimox-14 cationic surfactant. Biotechniques 15, 1102.

Hartel, C., Bein, G., Muller-Steinhardt, M. and Kluter, H. (2001) Ex vivo induction of cytokine mRNA expression in human blood samples. J Immunol Methods 249, 63.

Pradier, O., Surquin, M., Stordeur, P., De Pauw, L., Kinnaert, P., Vereerstraeten, P., Capel, P., Goldman, M. and Abramowicz, D. (1996) Monocyte procoagulant activity induced by in vivo administration of the OKT3 monoclonal antibody. Blood 87, 3768.

Schmidt, W.N., Klinzman, D., LaBrecque, D.R., Macfarlane, D.E. and Stapleton, J.T. (1995) Direct detection of hepatitis C virus (HCV) RNA from whole blood, and comparison with HCV RNA in plasma and peripheral blood mononuclear cells. J Med Virol 47, 153.

Stordeur, P., Schandene, L., Durez, P., Gerard, C., Goldman, M. and Velu, T. (1995) Spontaneous and cycloheximide-induced interleukin-10 mRNA expression in human mononuclear cells. Mol.Immunol. 32, 233.

Stordeur, P., Poulin, L.F., Craciun, L., Zhou, L., Schandené, L., de Lavareille, A., Goriely, S. and Goldman, M. Cytokine mRNA Quantification by Real Time PCR. J Immunol Methods, in press.

**TABLE 1. Oligonucleotides for real time PCR.**

| mRNA targets | Oligonucleotides (5'→3') * | Product size (bp) | Final concentration (nM) ** |
|---|---|---|---|
| IL-1ra | F264 : GAAGATGTGCCTGTCCTGTGT<br>R343 : CGCTCAGGTCAGTGATGTTAA<br>P291 : 6Fam-TGGTGATGAGACCAGACTCCAGCTG-Tamra-p | 80 | F 900<br>R 900 |
| IL-1β | F176 : ACAGATGAAGTGCTCCTTCCA<br>R248 : GTCGGAGATTCGTAGCTGGAT<br>P207 : 6Fam-CTCTGCCCTCTGGATGGCGG-Tamra-p | 73 | F 600<br>R 900 |
| IL-5 | F83 : AGCTGCCTACGTGTATGCCA<br>R153 : GCAGTGCCAAGGTCTCTTTCA<br>P104 : 6Fam-CCCCACAGAAATTCCCACAAGTGCATT-Tamra-p | 71 | F 300<br>R 900 |
| IL-10 | F409 : CATCGATTTCTTCCCTGTGAA<br>R482 : TCTTGGAGCTTATTAAAGGCATTC<br>P431 : 6Fam-ACAAGAGCAAGGCCGTGGAGCA-Tamra-p | 74 | F 600<br>R 900 |
| IL-13 | F155 : TGAGGAGCTGGTCAACATCA<br>R230 : CAGGTTGATGCTCCATACCAT<br>P187 : 6Fam-AGGCTCCGCTCTGCAATGGC-Tamra-p | 76 | F 900<br>R 900 |
| TNF-α | F275 : CCCAGGGACCTCTCTCTAATC<br>R358 : ATGGGCTACAGGCTTGTCACT<br>P303 : 6Fam-TGGCCCAGGCAGTCAGATCATC-Tamra-p | 84 | F 900<br>R 900 |
| IFN-γ | F464 : CTAATTATTCGGTAACTGACTTGA<br>R538 : ACAGTTCAGCCATCACTTGGA<br>P491 : 6Fam-TCCAACGCAAAGCAATACATGAAC-Tamra-p | 75 | F 600<br>R 900 |
| β-actin | F976 : GGATGCAGAAGGAGATCACTG<br>R1065 : CGATCCACACGGAGTACTTG<br>P997 : 6Fam-CCCTGGCACCCAGCACAATG-Tamra-p | 90 *** | F 300<br>R 300 |
| Mouse IL-9 (TaqMan probe) | F91 : GGCATCAGAGACACCAATTACCT<br>R233 : TGGCATTGGTCAGCTGTAACA<br>P184 : 6Fam-CTCTCCGTCCCAACTGATGATTGTACCAC-Tamra-p | 143 | F 300<br>R 300 |
| Mouse IL-9 (hybridisation probes) | F91 : GGCATCAGAGACACCAATTACCT<br>R233 : TGGCATTGGTCAGCTGTAACA<br>P163 : AACGTGACCAGCTGCTTGTGT-fluorescein<br>P185 : LCred 640-TCTCCGTCCCAACTGATGATT-p | 143 | F 300<br>R 900 |

* F, R and P indicate forward and reverse primers and probes, respectively; numbers indicate the sequence position.
** Final concentration of forward (F) and reverse (R) primers.
*** Except for IL-5, all primers were chosen to span intronic sequences so that genomic DNA amplification is not possible, excepted for β–actin for which a 112 bp longer band is obtained. If contaminating genomic DNA is detected using this size difference on agarose gel, a DNase digestion of all of the RNA samples coming from the same experiment is performed.

TABLE 2. Oligonucleotides for standard preparation.

| mRNA targets | Oligonucleotides (5'→3') * | Product size (bp) | Conditions for "classical" PCR ** |
|---|---|---|---|
| IL-1ra | F43 : CTCCTCTTCCTGTTCCATTC<br>R493 : CTTCGTCAGGCATATTGGT | 451 | A = 56 Mg = 1.5 |
| IL-1β | F59 : CTTCATTGCTCAAGTGTCTGAA<br>R553 : ACTTGTTGCTCCATATCCTGTC | 495 | A = 58 Mg = 1.5 |
| IL-10 | F296 : TTTACCTGGAGGAGGTGATG<br>R771 : TTGGGCTTCTTTCTAAATCGT | 476 | A = 56 Mg = 1.5 |
| IL-13 | F23 : GCTCCTCAATCCTCTCCTGT<br>R507 : GCAACTTCAATAGTCAGGTCCT | 485 | A = 56 Mg = 1.0 |
| TNF-α | F83 : ACCATGAGCACTGAAAGCAT<br>R488 : AGATGAGGTACAGGCCCTCT | 406 | A = 58 Mg = 1.5 |
| IFN-γ | F154 : TTGGGTTCTCTTGGCTGTTA<br>R632 : AAATATTGCAGGCAGGACAA | 479 | A = 58 Mg = 1.5 |
| β-actin | F745 : CCCTGGAGAAGAGCTACGA<br>R1253 : TAAAGCCATGCCAATCTCAT | 509 | A = 58 Mg = 1.5 |

* F and R indicate forward and reverse primers, respectively; numbers indicate the sequence position.

** Conditions, for all targets, were as follows: denaturation at 95 °C for 20 s, annealing (temperature as stated (A)) for 20 s and elongation at 72°C for 45 s, for a total of 35 cycles. $MgCl_2$ concentration (Mg, mM) was as stated. For the complete procedure, see (Stordeur et al., (1995), PCR for IFN-γ).

**TABLE 3. Coefficients of variation (CV) (%).**

A

| CV for mouse IL-9 **(n = 20)** | | Plasmid dilution (numbers of copies) | | |
|---|---|---|---|---|
| | | $1 \times 10^7$ | $1 \times 10^5$ | $1 \times 10^3$ |
| *Intra-run assays* | | | | |
| Lightcycler | Hybridisation probes | 8.81 | 7.41 | 8.78 |
| | TaqMan probe | 8.12 | 7.05 | 6.48 |
| GeneAmp 5700 | TaqMan probe | 6.95 | 8.83 | 11.49 |
| Inter-run assays | | | | |
| Lightcycler | Hybridisation probes | 21.95 | 12.91 | 30.61 |
| | TaqMan probe | 16.84 | 14.64 | 17.28 |
| GeneAmp 5700 | TaqMan probe | 31.16 | 20.32 | 40.71 |

B

| Intra-run CV for human IL-10 **(n = 20)** | **LPS-stimulated PBMC** * |
|---|---|
| | 6.94 |

* PBMC were stimulated for 6 h with 1 µg/ml LPS, and total RNA was extracted. Real time PCR for IL-10 was performed twenty times in one run, and the CV was calculated from absolute copy numbers.

TABLE 4.

| Comparison of Qiagen and MagNA Pure LC extraction methods. | |
|---|---|
| **4.1. Qiagen mRNA extraction method. Blood mRNA coming from the same blood sample was extracted 9 times.** | |
| | **IFN-γ mRNA copy numbers per million of β-actin mRNA copies** |
| result 1 | 35 |
| result 2 | 25 |
| result 3 | 29 |
| result 4 | 27 |
| result 5 | 27 |
| result 6 | 49 |
| result 7 | 33 |
| result 8 | 22 |
| result 9 | 27 |
| *mean* | *30* |
| *SD* | *8* |
| ***CV*** | ***26*** |
| **4.2. MagNA Pure LC (kit + instrument) mRNA extraction method. Blood mRNA prepared from the same blood sample was extracted 9 times.** | |
| | **IFN-γ mRNA copy numbers per million of β-actin mRNA copies** |
| result 1 | 192 |
| result 2 | 170 |
| result 3 | 153 |
| result 4 | 139 |
| result 5 | 138 |
| result 6 | 160 |
| result 7 | 105 |
| result 8 | 142 |
| result 9 | 142 |
| *mean* | *149* |
| *SD* | *24* |
| ***CV*** | ***16*** |

## Claims

**1.** A method for the quantification of *in vivo* RNA from a biological sample comprising the steps of:

(a) collecting said biological sample in a tube comprising a compound inhibiting RNA degradation and/or gene induction,
(b) forming a precipitate comprising nucleic acids,
(c) separating said precipitate of step (b) from the supernatant,
(d) dissolving said precipitate of step (c) using a buffer, forming a suspension,
(e) isolating nucleic acids from said suspension of step (d) using an automated device,
(f) dispersing/distributing a reagent mix for RT-PCR using an automated device,
(g) dispersing/distributing the nucleic acids isolated in step (e) within the dispersed reagent mix of step (f) using an automated device, and,
(h) determining the *in vivo* levels of transcripts using the nucleic acid/RT-PCR reagent mix of step (g) in an

automated setup.

**2.** A method according to claim 1, whereby steps (a) and (b) are performed simultaneously.

**3.** A method according to claim 1 or 2, whereby said compound of step (a) comprises a quaternary amine surfactant.

**4.** A method according to claim 3, whereby said quaternary amine is tetradecyltrimethylammonium oxalate.

**5.** A method according to claim 1 or 2, whereby said compound of step (a) is a compound inhibiting cellular RNA degradation and/or gene induction as found in a PAXgene™ Blood RNA Tube.

**6.** A method according to any of the claims 1 to 5, whereby said tube of step (a) is an open or a closed blood collecting tube.

**7.** A method according to any of the claims 1 to 6, whereby said buffer of step (d) is a guanidine-thiocyanate-containing buffer.

**8.** A method according to claim 7, whereby said guanidine-thiocyanate-containing buffer is a lysis buffer as provided by the MagNA Pure LC mRNA Isolation Kit I (Roche Diagnostics, Molecular Biochemicals).

**9.** A method according to any of the claims 1 to 8, whereby said isolation of nucleic acids of step (e) is performed using RNA-capturing beads.

**10.** A method according to any of the claims 1 to 9, whereby said automated device of step (e), step (f) and/or step (g) is the MagNA Pure LC Instrument (Roche Diagnostics, Molecular Biochemicals).

**11.** A method according to any of the claims 1 to 10, whereby said *in vivo* levels are determined using real time PCR.

**12.** A method according to any of the claims 1 to 11, whereby said quantification is performed using a biological sample of 100 µl.

**13.** A method for the quantification of *in vivo* RNA from a biological sample comprising the steps of:

(a) collecting a biological sample in the PAXgene™ RNA Tube,
(b) dissociating the surfactant/nucleic acid complex with a guanidine isothiocyanate buffer,
(c) extracting mRNA and/or total RNA using an reproducible automated device,
(d) dispersing/distributing a reagent mix for RT-PCR using an automated device,
(e) dispersing/distributing the nucleic acids isolated in step (c) within the dispersed reagent mix of step (d) using an automated device, and,
(f) quantifying RNA by real time PCR in an automated setup, whereby the RT and the PCR reaction are performed in one step.

**14.** A kit for isolating quantifiable *in vivo* RNA from a biological sample comprising:

(a) optionally, a collection tube for biological samples,
(b) a compound inhibiting RNA degradation and/or gene induction,
(c) reagents for automated RNA isolation,
(d) a reagent mix for a simultaneous RT and real-time PCR reaction or separate compounds thereof , allowing the automated dispersion of said mix,
(e) optionally, specific oligonucleotides to perform said RT-PCT reactions, and,
(f) optionally, an instruction manual describing a method for an automated RNA isolation, a method for the automated dispersion of a reagent mix and the dispersion of the isolated nucleic acids for RT- real time PCR, and a method for automated RNA analysis.

**15.** A kit according to claim 14, wherein said compound of part (b) is a compound as defined in any of the methods of claims 3 to 5.

**16.** A kit according to claims 14 and 15, wherein additionally a buffer is provided as defined in any of the methods of

claims 7 to 8.

**17.** A kit for isolating quantifiable *in vivo* RNA from a biological sample comprising:

(a) a PAXgene™ Blood RNA Tube,
(b) a guanidine isothiocyanate buffer,
(c) reagents for automated RNA isolation,
(d) a reagent mix for a simultaneous RT and real-time PCR reaction or separate compounds thereof , allowing the automated dispersion of said mix,
(e) optionally, specific oligonucleotides to perform said RT-PCT reactions, and,
(f) optionally, an instruction manual describing a method for an automated RNA isolation, a method for the automated dispersion of a reagent mix and the dispersion of the isolated nucleic acids for RT- real time PCR, and a method for automated RNA analysis.

**18.** A method for the quantification of DNA from a biological sample wherein a method is used as performed for the quantification of RNA according to the methods of any of claims 1 to 13 wherein the RT reaction is skipped and wherein the compound of step

(a) also protects the DNA from being degraded.

**19.** A kit for isolating quantifiable DNA from a biological sample according to any of the claims 14 to 17, wherein a reagent mix/compounds for performing said RT reaction is absent.

**20.** Use of a method according to any of the claims 1 to 13 or claim 18 or a kit according to any of the claims 14 to 17 or claim 19 for the monitoring/detection of changes of *in vivo* nucleic acids levels in a biological agent present in a biological sample.

**21.** Use of a method or a kit according to claim 20 whereby said biological agent is chosen from a group consisting of eukaryotic cells, prokaryotic cells, viruses and phages.

**22.** Use of a method according to any of the claims 1 to 13 or claim 18 or a kit according to any of the claims 14 to 17 or claim 19 for the monitoring/detection of changes of *in vivo* nucleic acids of a biological agent in a biological sample, in order to diagnose a certain disease.

**23.** Use of a method according to any of the claims 1 to 13 or claim 18 or a kit according to any of the claims 14 to 17 or claim 19 for the monitoring/detection of changes of *in vivo* nucleic acids of a biological agent in a biological sample, in order to screen for a compound for the production of a medicament for curing a disease.

**24.** A compound identifiable by a method according to claim 23.

**25.** Use of a method or a kit according to claim 22 and/or 23, wherein said disease is an immuno-related disease.

**26.** Use of a method or a kit according to claim 23, for the detection/monitoring/screening of a compound, wherein said compound is an immunomodulatory compound which may be chosen from a group consisting of eukaryotic cells, prokaryotic cells, viruses, phages, parasites, drugs (natural extracts, organic molecule, peptide, proteins, nucleic acids), medical treatment, vaccine and transplants.

**27.** Use of a method according to any of the claims 1 to 13 or claim 18 or a kit according to any of the claims 14 to 17 or claim 19, for the detection/monitoring of epitope specific CTLs or immuno-related transcripts.

**28.** A method to identify an agent capable of modifying the immunological status of a subject via the analysis of epitope specific CTLs comprising the steps of:

(a) applying an immunomodulatory agent(s) into a subject,
(b) sampling whole blood from said subject,
(c) optionally, pulsing blood cells present in the whole blood sample of step (b) with an identical/ similar and/ or different immunomodulatory agent as applied in step (a),
(d) collecting pulsed blood cells of step (c) or non-pulsed blood cells of step (b) in a tube comprising a compound

inhibiting RNA degradation and/or gene induction, or adding said compound to the pulsed/ non-pulsed cells,
(e) forming a precipitate comprising nucleic acids,
(f) separating said precipitate of step (e) from the supernatant,
(g) dissolving said precipitate of step (f) using a buffer, forming a suspension,
(h) isolating nucleic acids from said suspension of step (g) using an automated device,
(i) dispersing/distributing a reagent mix for RT-PCR using an automated device,
(j) dispersing/distributing the nucleic acids isolated in step (h) within the dispersed reagent mix of step (i) using an automated device,
(k) detecting/ monitoring/ analyzing the *in vivo* levels of epitope specific CTLs-related transcripts in the dispersed solution of step (j) in an automated setup, and,
(l) identify agents able to modify the immunological status of said subject, whereby, in case the agent of step (a) is already present in the subject, step (a) is omitted.

**29.** A method to identify an agent capable of modifying the immunological status of a subject:

(a) applying an immunomodulatory agent(s) into a subject,
(b) sampling whole blood from said subject,
(c) optionally, pulsing blood cells present in the whole blood sample of step (b) with an identical/ similar and/or different immunomodulatory agent as applied in step (a),
(d) collecting pulsed blood cells of step (c) or non-pulsed blood cells of step (b) in a tube comprising a compound inhibiting RNA degradation and/or gene induction, or adding said compound to the pulsed/non-pulsed cells,
(e) forming a precipitate comprising nucleic acids,
(f) separating said precipitate of step (e) from the supernatant,
(g) dissolving said precipitate of step (f) using a buffer, forming a suspension,
(h) isolating nucleic acids from said suspension of step (g) using an automated device,
(i) dispersing/distributing a reagent mix for RT-PCR using an automated device,
(j) dispersing/distributing the nucleic acids isolated in step (h) within the dispersed reagent mix of step (i) using an automated device,
(k) detecting/ monitoring/ analyzing the *in vivo* levels of immuno-related transcripts in the dispersed solution of step (j) in an automated setup, and,
(m) identify agents able to modify the immunological status of said subject,
whereby, in case the agent of step (a) is already present in the subject, step (a) is omitted.

**30.** A method for the diagnosis/ prognosis/ monitoring of a clinical status affecting the immune system in a subject comprising the steps of:

(a) sampling whole blood from said subject in a tube comprising a compound inhibiting
RNA degradation and/or gene induction, or adding said compound to the blood cells,
(b) forming a precipitate comprising nucleic acids,
(c) separating said precipitate of step (b) from the supernatant,
(d) dissolving said precipitate of step (c) using a buffer, forming a suspension,
(e) isolating nucleic acids from said suspension of step (d) using an automated device,
(f) dispersing/distributing a reagent mix for RT-PCR using an automated device,
(g) dispersing/distributing the nucleic acids isolated in step (e) within the dispersed reagent mix of step (f) using an automated device,
(h) detecting/ monitoring/ analyzing the *in vivo* levels of immuno-related transcripts in the dispersed solution of step (g) in an automated setup, and,
(i) detecting/ monitoring the change in *in vivo* levels of immuno-related transcripts, and,
(j) diagnosing/ prognosing/ monitoring the disease affecting the immune system.

**31.** A method for the diagnosis/ prognosis/ monitoring of a clinical status affecting the immune system in a subject comprising the steps of:

(a) sampling whole blood from said subject,
(b) pulsing blood cells present in the whole blood sample of step (a) with an identical/ similar and/or different immunomodulatory agent as present in the subject,
(c) collecting pulsed blood cells of step (b) in a tube comprising a compound inhibiting
RNA degradation and/or gene induction, or adding said compound to the pulsed cells,

(d) forming a precipitate comprising nucleic acids,
(e) separating said precipitate of step (d) from the supernatant,
(f) dissolving said precipitate of step (e) using a buffer, forming a suspension,
(g) isolating nucleic acids from said suspension of step (f) using an automated device,
(h) dispersing/distributing a reagent mix for RT-PCR using an automated device,
(i) dispersing/distributing the nucleic acids isolated in step (g) within the dispersed reagent mix of step (h) using an automated device,
(j) detecting/ monitoring/ analyzing the *in vivo* levels of immuno-related transcripts in the dispersed solution of step (i) in an automated setup,
(k) detecting/ monitoring the change in *in vivo* levels of immuno-related transcripts, and,
(l) diagnosing/ prognosing/ monitoring the disease affecting the immune system.

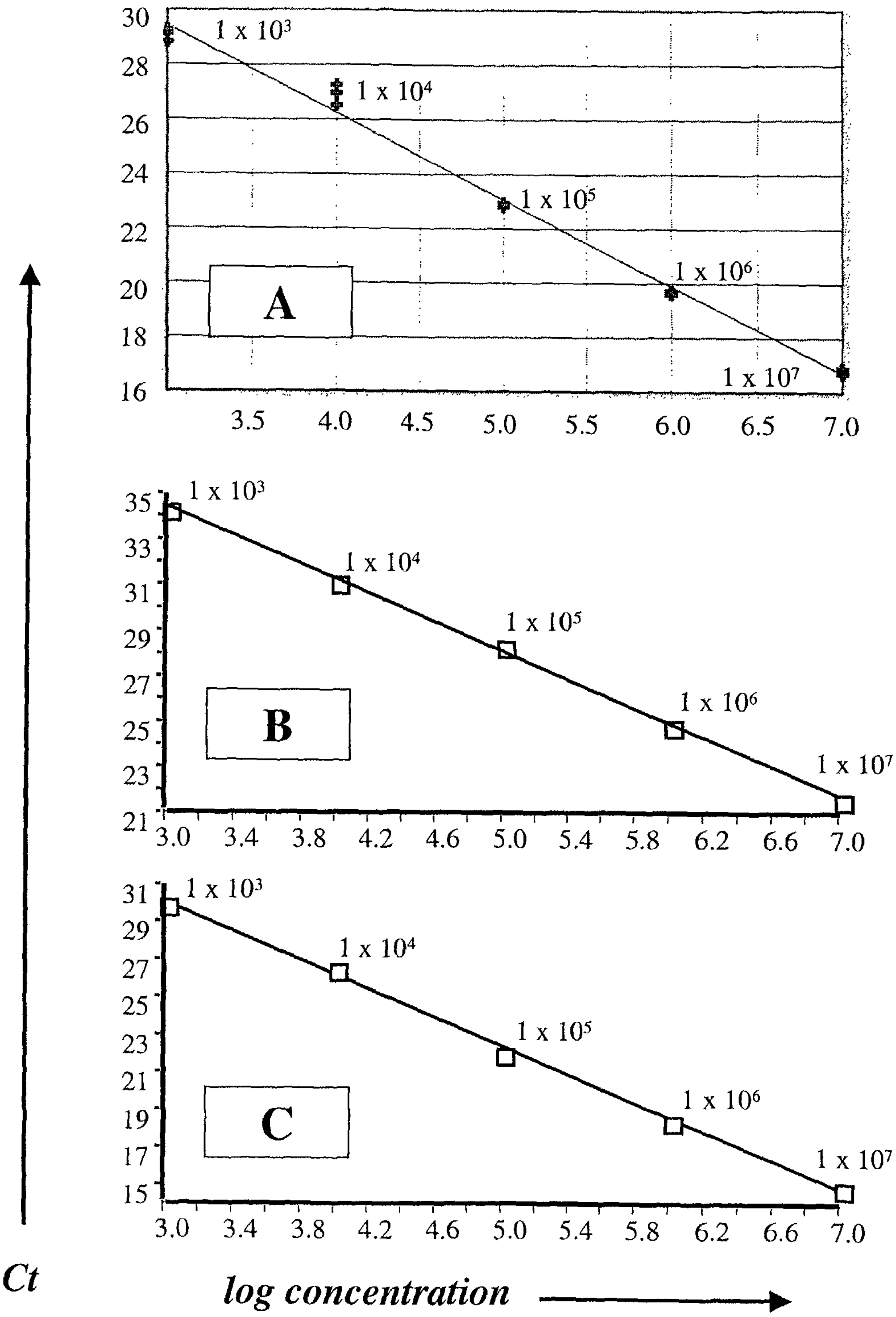
30
28
26
24
22
20
18
16
1 x 10³
1 x 10⁴
1 x 10⁵
1 x 10⁶
1 x 10⁷
A
3.5 4.0 4.5 5.0 5.5 6.0 6.5 7.0
35 33 31 29 27 25 23 21
B
3.0 3.4 3.8 4.2 4.6 5.0 5.4 5.8 6.2 6.6 7.0
31 29 27 25 23 21 19 17 15
C
Ct
log concentration

figure 1.1

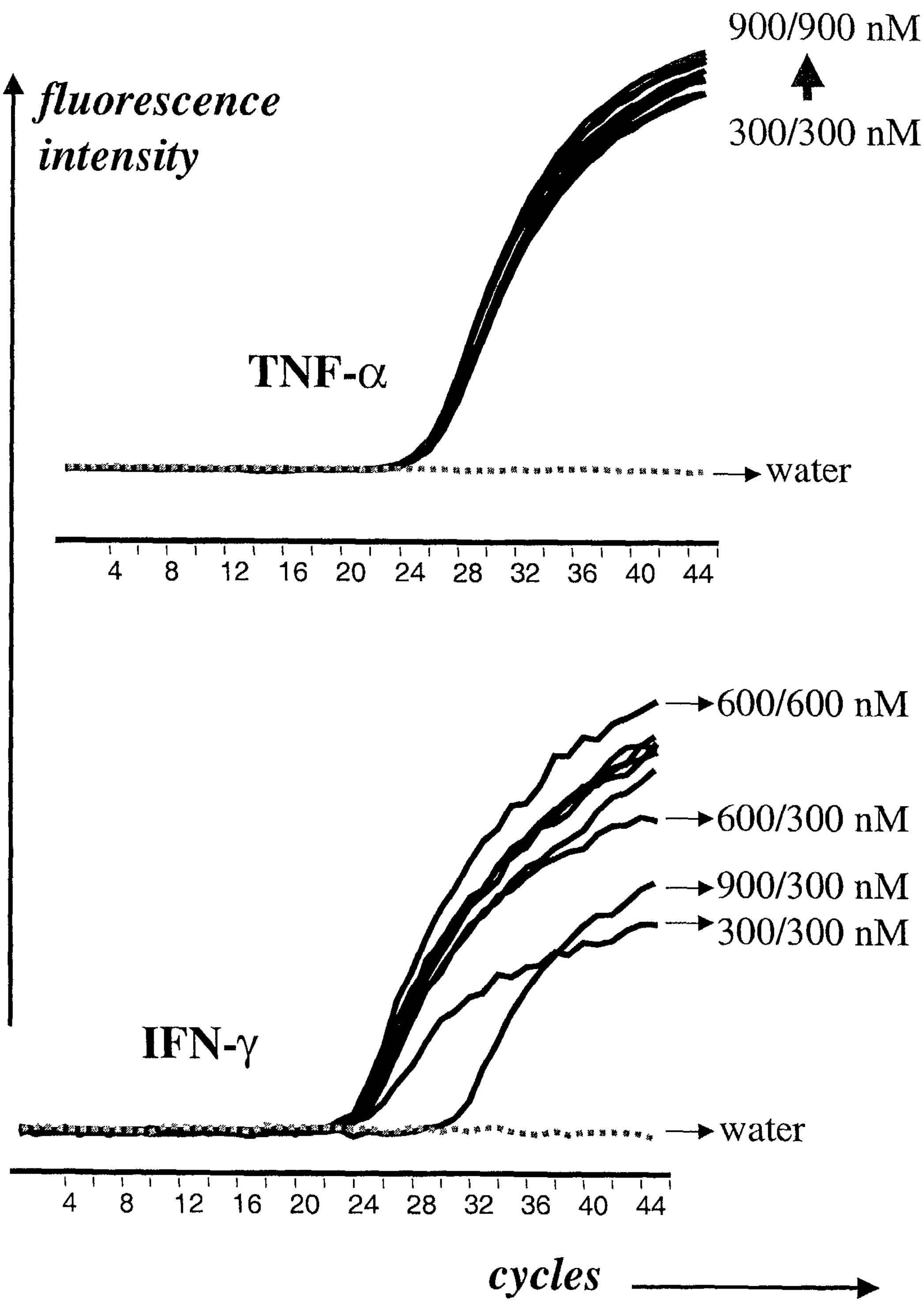
900/900 nM
300/300 nM
fluorescence intensity
TNF-α
water
4 8 12 16 20 24 28 32 36 40 44
600/600 nM
600/300 nM
900/300 nM
300/300 nM
IFN-γ
water
4 8 12 16 20 24 28 32 36 40 44
cycles

figure 1.2

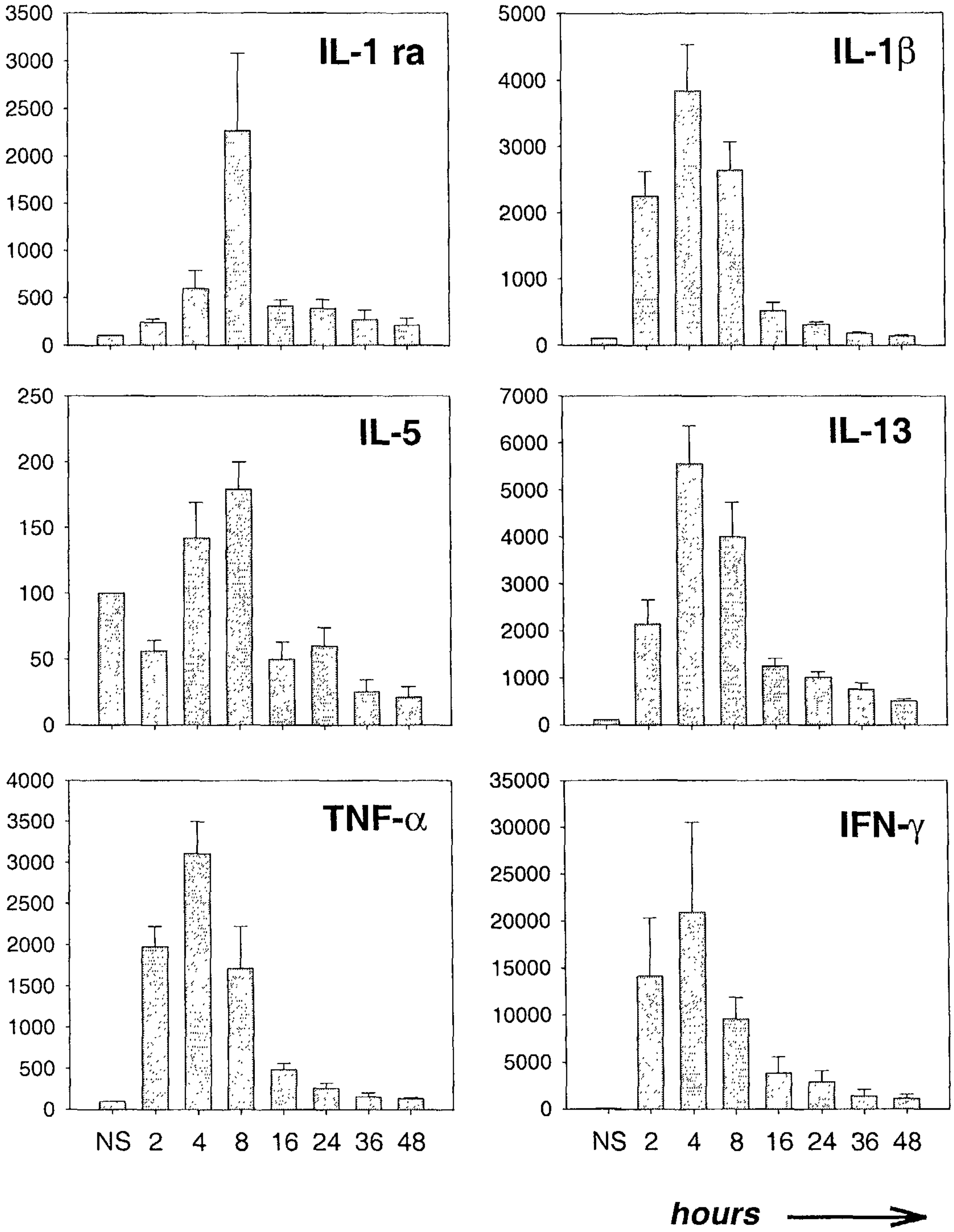
IL-1 ra
3500
3000
2500
2000
1500
1000
500
0
IL-1β
5000
4000
3000
2000
1000
0
IL-5
250
200
150
100
50
0
IL-13
7000
6000
5000
4000
3000
2000
1000
0
TNF-α
4000
3500
3000
2500
2000
1500
1000
500
0
IFN-γ
35000
30000
25000
20000
15000
10000
5000
0
NS 2 4 8 16 24 36 48
NS 2 4 8 16 24 36 48
hours

figure 1.3

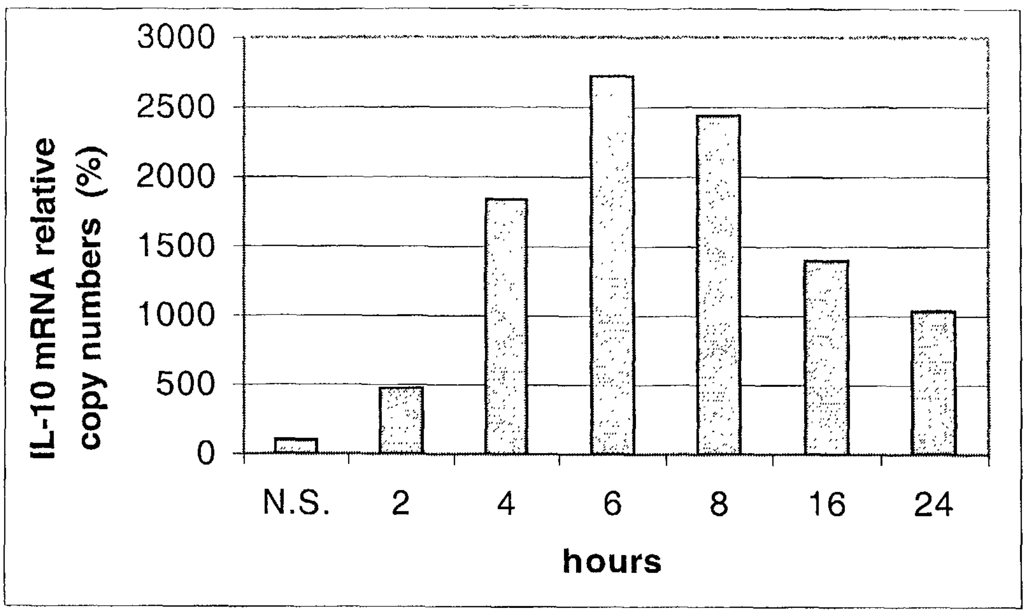
IL-10 mRNA relative copy numbers (%)
3000
2500
2000
1500
1000
500
0
N.S.
2
4
6
8
16
24
hours

figure 1.4

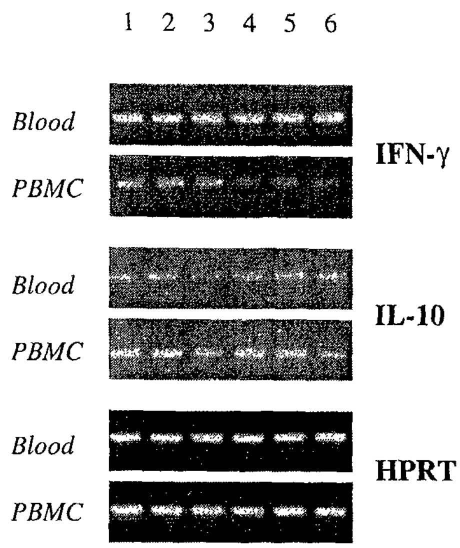


Figure 2.1

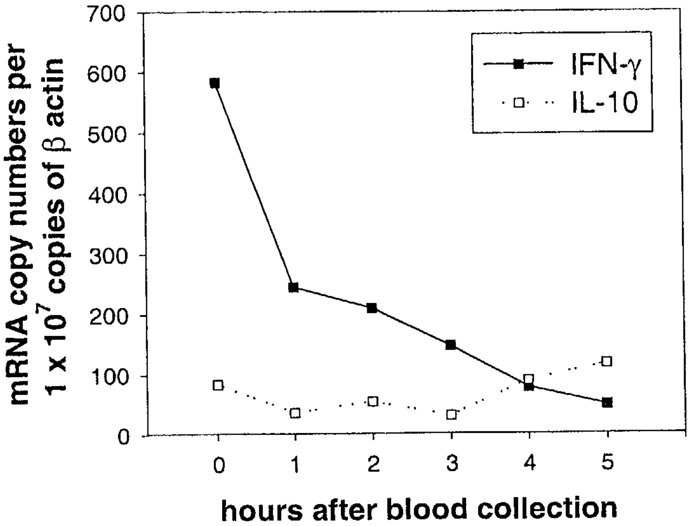
700
600
500
400
300
200
100
0
mRNA copy numbers per
1 x 10$^7$ copies of β actin
IFN-γ
IL-10
0
1
2
3
4
5
hours after blood collection

Figure 2.2

Figure 3:

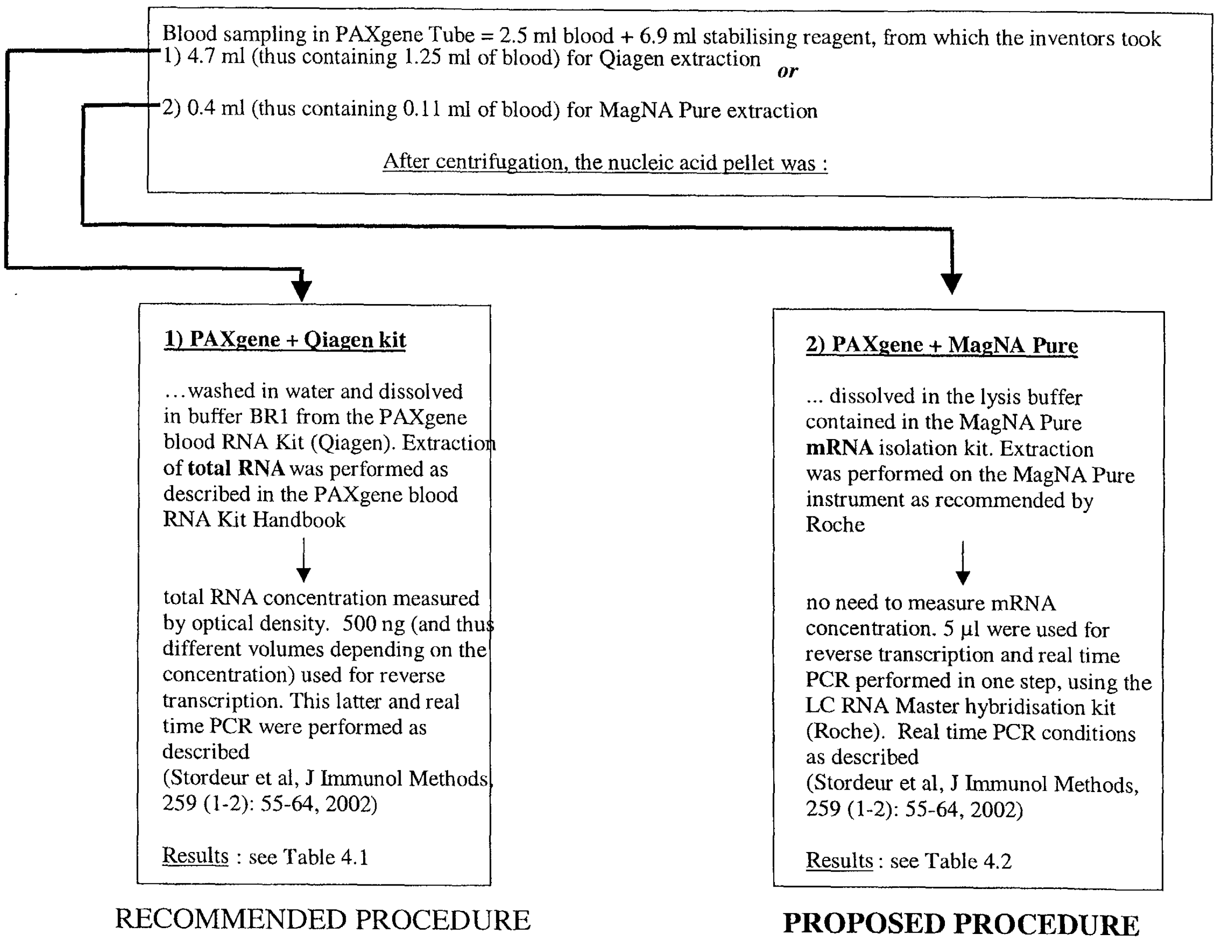
Blood sampling in PAXgene Tube = 2.5 ml blood + 6.9 ml stabilising reagent, from which the inventors took
1) 4.7 ml (thus containing 1.25 ml of blood) for Qiagen extraction
or
2) 0.4 ml (thus containing 0.11 ml of blood) for MagNA Pure extraction
After centrifugation, the nucleic acid pellet was :
1) PAXgene + Qiagen kit
...washed in water and dissolved in buffer BR1 from the PAXgene blood RNA Kit (Qiagen). Extraction of total RNA was performed as described in the PAXgene blood RNA Kit Handbook
total RNA concentration measured by optical density. 500 ng (and thus different volumes depending on the concentration) used for reverse transcription. This latter and real time PCR were performed as described (Stordeur et al, J Immunol Methods, 259 (1-2): 55-64, 2002)
Results : see Table 4.1
RECOMMENDED PROCEDURE
2) PAXgene + MagNA Pure
... dissolved in the lysis buffer contained in the MagNA Pure mRNA isolation kit. Extraction was performed on the MagNA Pure instrument as recommended by Roche
no need to measure mRNA concentration. 5 µl were used for reverse transcription and real time PCR performed in one step, using the LC RNA Master hybridisation kit (Roche). Real time PCR conditions as described (Stordeur et al, J Immunol Methods, 259 (1-2): 55-64, 2002)
Results : see Table 4.2
PROPOSED PROCEDURE

Figure 4

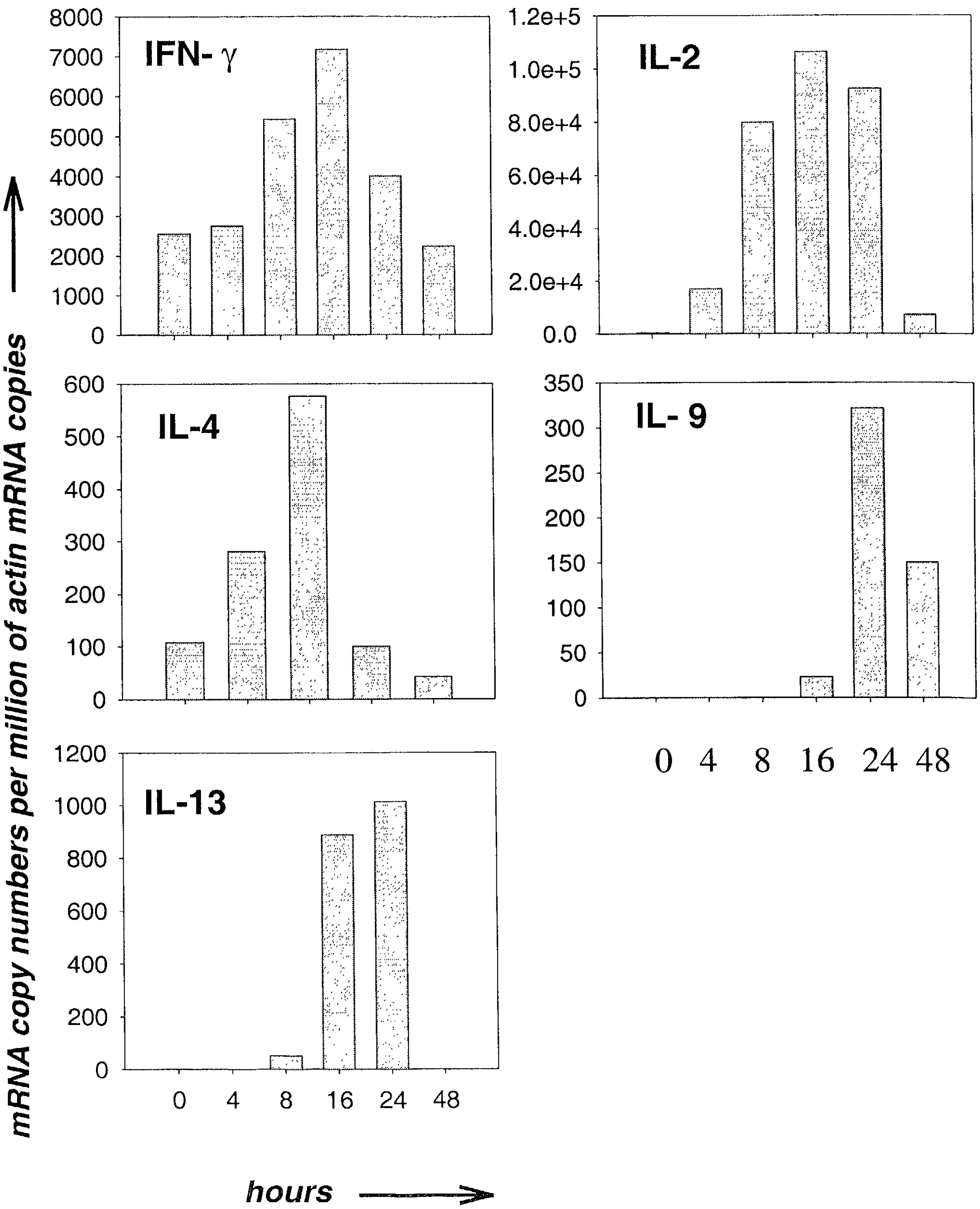
IFN-γ
IL-2
IL-4
IL-9
IL-13
mRNA copy numbers per million of actin mRNA copies
hours
0 4 8 16 24 48

European Patent Office

# EUROPEAN SEARCH REPORT

Application Number
EP 02 44 7009

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 02 00599 A (QIAGEN GMBH ;HOLLAENDER VERA (DE); OELMUELLER UWE (DE); WYRICH RAL) 3 January 2002 (2002-01-03) * the whole document * | 1-13 | C12N15/10 C12Q1/68 |
| X | WO 94 18156 A (UNIV IOWA RES FOUND) 18 August 1994 (1994-08-18) * claims 1-12; example 1 * | 1-13 | |
| D,X | US 5 906 744 A (CARROLL RICHARD J ET AL) 25 May 1999 (1999-05-25) * claim 1 * | 5,13 | |

TECHNICAL FIELDS SEARCHED (Int.Cl.7)
C12N
C12Q

The present search report has been drawn up for all claims

| Place of search | Date of completion of the search | Examiner |
|---|---|---|
| THE HAGUE | 3 September 2002 | Gabriels, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03 82 (P04C01)

## ANNEX TO THE EUROPEAN SEARCH REPORT ON EUROPEAN PATENT APPLICATION NO. EP 02 44 7009

This annex lists the patent family membersrelating to the patent documents cited in the above-mentioned European search report.
The members are as contained in the European Patent Office EDP file on
The European Patent Office is in no way liable for these particulars which are merely given for the purpose of information.

03-09-2002

| Patent document cited in search report | Publication date | Patent family member(s) | Publication date |
|---|---|---|---|
| WO 0200599 A | 03-01-2002 | DE 10031236 A1 | 10-01-2002 |
| | | AU 6903101 A | 08-01-2002 |
| | | AU 7568501 A | 08-01-2002 |
| | | WO 0200599 A1 | 03-01-2002 |
| | | WO 0200600 A1 | 03-01-2002 |
| WO 9418156 A | 18-08-1994 | US 5300635 A | 05-04-1994 |
| | | AU 6230594 A | 29-08-1994 |
| | | JP 8506340 T | 09-07-1996 |
| | | US 5985572 A | 16-11-1999 |
| | | WO 9418156 A1 | 18-08-1994 |
| | | US 5728822 A | 17-03-1998 |
| US 5906744 A | 25-05-1999 | AU 738911 B2 | 27-09-2001 |
| | | AU 6360098 A | 05-11-1998 |
| | | BR 9800776 A | 07-12-1999 |
| | | DE 875757 T1 | 02-06-1999 |
| | | EP 0875757 A2 | 04-11-1998 |
| | | JP 11118791 A | 30-04-1999 |

EPO FORM P0459

For more details about this annex : see Official Journal of the European Patent Office, No. 12/82

SEQUENCE LISTING

<110> CYPRO S.A.

<120> Method to determine in vivo nucleic acid levels

<130> ULB-009-EP

<140> EP 02447009.8
<141> 2002-01-18

<160> 45

<170> PatentIn version 3.1

<210> 1
<211> 21
<212> DNA
<213> Artificial Sequence

<220>
<223> Oligonucleotide

<400> 1
gaagatgtgc ctgtcctgtg t 21

<210> 2
<211> 21
<212> DNA
<213> Artificial Sequence

<220>
<223> Oligonucleotide

<400> 2
cgctcaggtc agtgatgtta a 21

<210> 3
<211> 27
<212> DNA
<213> Artificial Sequence

<220>
<223> Oligonucleotide

<220>
<221> misc_feature
<222> (1)..(1)
<223> N = 6Fam

<220>
<221> misc_feature
<222> (27)..(27)
<223> N = Tamra-p

<400> 3
ntggtgatga gaccagactc cagctgn 27

<210> 4
<211> 21
<212> DNA
<213> Artificial Sequence

<220>
<223> Oligonucleotide

<400> 4
acagatgaag tgctccttcc a 21

<210> 5
<211> 21
<212> DNA
<213> Artificial Sequence

<220>
<223> Oligonucleotide

<400> 5
gtcggagatt cgtagctgga t 21

<210> 6
<211> 22
<212> DNA
<213> Artificial Sequence

<220>
<223> Oligonucleotide

<220>
<221> misc_feature
<222> (1)..(1)
<223> N = 6Fam

<220>
<221> misc_feature
<222> (22)..(22)
<223> N = Tamra-p

<400> 6
nctctgccct ctggatggcg gn 22

<210> 7
<211> 20
<212> DNA
<213> Artificial Sequence

<220>
<223> Oligonucleotide

<400> 7
agctgcctac gtgtatgcca 20

<210> 8
<211> 21
<212> DNA

<213> Artificial Sequence

<220>
<223> Oligonucleotide

<400> 8
gcagtgccaa ggtctctttc a 21

<210> 9
<211> 29
<212> DNA
<213> Artificial Sequence

<220>
<223> Oligonucleotide

<220>
<221> misc_feature
<222> (1)..(1)
<223> N = 6Fam

<220>
<221> misc_feature
<222> (29)..(29)
<223> N = Tamra-p

<400> 9
nccccacaga aattcccaca agtgcattn 29

<210> 10
<211> 21
<212> DNA
<213> Artificial Sequence

<220>
<223> Oligonucleotide

<400> 10
catcgatttc ttccctgtga a 21

<210> 11
<211> 24
<212> DNA
<213> Artificial Sequence

<220>
<223> Oligonucleotide

<400> 11
tcttggagct tattaaaggc attc 24

<210> 12
<211> 24
<212> DNA
<213> Artificial Sequence

<220>

<223> Oligonucleotide

<220>
<221> misc_feature
<222> (1)..(1)
<223> N = 6Fam

<220>
<221> misc_feature
<222> (24)..(24)
<223> N = Tamra-p

<400> 12
nacaagagca aggccgtgga gcan 24

<210> 13
<211> 20
<212> DNA
<213> Artificial Sequence

<220>
<223> Oligonucleotide

<400> 13
tgaggagctg gtcaacatca 20

<210> 14
<211> 21
<212> DNA
<213> Artificial Sequence

<220>
<223> Oligonucleotide

<400> 14
caggttgatg ctccatacca t 21

<210> 15
<211> 22
<212> DNA
<213> Artificial Sequence

<220>
<223> Oligonucleotide

<220>
<221> misc_feature
<222> (1)..(1)
<223> N = 6FAM

<220>
<221> misc_feature
<222> (22)..(22)
<223> N = Tamra-p

<400> 15

naggctccgc tctgcaatgg cn 22

<210> 16
<211> 21
<212> DNA
<213> Artificial Sequence

<220>
<223> Oligonucleotide

<400> 16
cccagggacc tctctctaat c 21

<210> 17
<211> 21
<212> DNA
<213> Artificial Sequence

<220>
<223> Oligonucleotide

<400> 17
atgggctaca ggcttgtcac t 21

<210> 18
<211> 24
<212> DNA
<213> Artificial Sequence

<220>
<223> Oligonucleotide

<220>
<221> misc_feature
<222> (1)..(1)
<223> N = 6Fam

<220>
<221> misc_feature
<222> (24)..(24)
<223> N = Tamra-p

<400> 18
ntggcccagg cagtcagatc atcn 24

<210> 19
<211> 24
<212> DNA
<213> Artificial Sequence

<220>
<223> Oligonucleotide

<400> 19
ctaattattc ggtaactgac ttga 24

<210> 20
<211> 21
<212> DNA
<213> Artificial Sequence

<220>
<223> Oligonucleotide

<400> 20
acagttcagc catcacttgg a 21

<210> 21
<211> 26
<212> DNA
<213> Artificial Sequence

<220>
<223> Oligonucleotide

<220>
<221> misc_feature
<222> (1)..(1)
<223> N = 6Fam

<220>
<221> misc_feature
<222> (26)..(26)
<223> N = Tamra-p

<400> 21
ntccaacgca aagcaataca tgaacn 26

<210> 22
<211> 21
<212> DNA
<213> Artificial Sequence

<220>
<223> Oligonucleotide

<400> 22
ggatgcagaa ggagatcact g 21

<210> 23
<211> 20
<212> DNA
<213> Artificial Sequence

<220>
<223> Oligonucleotide

<400> 23
cgatccacac ggagtacttg 20

<210> 24
<211> 22
<212> DNA

<213>  Artificial Sequence

<220>
<223>  Oligonucleotide

<220>
<221>  misc_feature
<222>  (1)..(1)
<223>  N = 6Fam

<220>
<221>  misc_feature
<222>  (22)..(22)
<223>  N = Tamra-p

<400>  24
nccctggcac ccagcacaat gn    22

<210>  25
<211>  23
<212>  DNA
<213>  Artificial Sequence

<220>
<223>  Oligonucleotide

<400>  25
ggcatcagag acaccaatta cct    23

<210>  26
<211>  21
<212>  DNA
<213>  Artificial Sequence

<220>
<223>  Oligonucleotide

<400>  26
tggcattggt cagctgtaac a    21

<210>  27
<211>  31
<212>  DNA
<213>  Artificial Sequence

<220>
<223>  Oligonucleotide

<220>
<221>  misc_feature
<222>  (1)..(1)
<223>  N = 6Fam

<220>
<221>  misc_feature
<222>  (31)..(31)
<223>  N = Tamra-p

<400> 27
nctctccgtc ccaactgatg attgtaccac n 31

<210> 28
<211> 23
<212> DNA
<213> Artificial Sequence

<220>
<223> Oligonucleotide

<400> 28
ggcatcagag acaccaatta cct 23

<210> 29
<211> 21
<212> DNA
<213> Artificial Sequence

<220>
<223> Oligonucleotide

<400> 29
tggcattggt cagctgtaac a 21

<210> 30
<211> 22
<212> DNA
<213> Artificial Sequence

<220>
<223> Oligonucleotide

<220>
<221> misc_feature
<222> (22)..(22)
<223> N = fluorescein

<400> 30
aacgtgacca gctgcttgtg tn 22

<210> 31
<211> 23
<212> DNA
<213> Artificial Sequence

<220>
<223> Oligonucleotide

<220>
<221> misc_feature
<222> (1)..(1)
<223> N = LCred 640

<220>

<221> misc_feature
<222> (23)..(23)
<223> N = p

<400> 31
ntctccgtcc caactgatga ttn 23

<210> 32
<211> 20
<212> DNA
<213> Artificial Sequence

<220>
<223> Oligonucleotide

<400> 32
ctcctcttcc tgttccattc 20

<210> 33
<211> 19
<212> DNA
<213> Artificial Sequence

<220>
<223> Oligonucleotide

<400> 33
cttcgtcagg catattggt 19

<210> 34
<211> 22
<212> DNA
<213> Artificial Sequence

<220>
<223> Oligonucleotide

<400> 34
cttcattgct caagtgtctg aa 22

<210> 35
<211> 22
<212> DNA
<213> Artificial Sequence

<220>
<223> Oligonucleotide

<400> 35
acttgttgct ccatatcctg tc 22

<210> 36
<211> 20
<212> DNA
<213> Artificial Sequence

<220>

<223> Oligonucleotide

<400> 36
tttacctgga ggaggtgatg 20

<210> 37
<211> 21
<212> DNA
<213> Artificial Sequence

<220>
<223> Oligonucleotide

<400> 37
ttgggcttct ttctaaatcg t 21

<210> 38
<211> 20
<212> DNA
<213> Artificial Sequence

<220>
<223> Oligonucleotide

<400> 38
gctcctcaat cctctcctgt 20

<210> 39
<211> 22
<212> DNA
<213> Artificial Sequence

<220>
<223> Oligonucleotide

<400> 39
gcaacttcaa tagtcaggtc ct 22

<210> 40
<211> 20
<212> DNA
<213> Artificial Sequence

<220>
<223> Oligonucleotide

<400> 40
accatgagca ctgaaagcat 20

<210> 41
<211> 20
<212> DNA
<213> Artificial Sequence

<220>
<223> Oligonucleotide

<400> 41

```
agatgaggta caggccctct                                                  20


<210>  42
<211>  20
<212>  DNA
<213>  Artificial Sequence

<220>
<223>  Oligonucleotide

<400>  42
ttgggttctc ttggctgtta                                                  20


<210>  43
<211>  20
<212>  DNA
<213>  Artificial Sequence

<220>
<223>  Oligonucleotide

<400>  43
aaatattgca ggcaggacaa                                                  20


<210>  44
<211>  19
<212>  DNA
<213>  Artificial Sequence

<220>
<223>  Oligonucleotide

<400>  44
ccctggagaa gagctacga                                                   19


<210>  45
<211>  20
<212>  DNA
<213>  Artificial Sequence

<220>
<223>  Oligonucleotide

<400>  45
taaagccatg ccaatctcat                                                  20
```